(19) Europäisches Patentamt
European Patent Office
Office européen des brevets

(11) EP 1 497 304 B1

(12) **EUROPEAN PATENT SPECIFICATION**

(45) Date of publication and mention of the grant of the patent:
**25.06.2014 Bulletin 2014/26**

(21) Application number: **03721530.8**

(22) Date of filing: **04.04.2003**

(51) Int Cl.:
*C07H 21/00* (2006.01) *C07H 21/02* (2006.01)
*C07H 21/04* (2006.01) *C12Q 1/68* (2006.01)

(86) International application number:
**PCT/US2003/010306**

(87) International publication number:
**WO 2003/087302 (23.10.2003 Gazette 2003/43)**

# (54) DUAL-LABELED NUCLEOTIDES

DOPPELT MARKIERTE NUKLEOTIDE

NUCLEOTIDES A DOUBLE MARQUAGE

(84) Designated Contracting States:
**AT BE BG CH CY CZ DE DK EE ES FI FR GB GR HU IE IT LI LU MC NL PT RO SE SI SK TR**

(30) Priority: **12.04.2002 US 372351 P**
**02.08.2002 US 400558 P**

(43) Date of publication of application:
**19.01.2005 Bulletin 2005/03**

(73) Proprietor: **Catalyst Assets LLC**
**Jackson, WY 83001 (US)**

(72) Inventors:
- **ANDERSON, Jack, D.**
  **Oceanside, CA 92057 (US)**
- **BRAMAN, Jeffrey, Carl**
  **Carlsbad, CA 92009 (US)**

(74) Representative: **Lloyd, John Scott et al**
**Maschio & Soames IP Limited**
**20 Carlton Crescent**
**Southampton, SO15 2ET (GB)**

(56) References cited:
**US-A1- 2001 018 184** **US-B1- 6 255 083**

- **NOETH: "The mini Quick Spin Column: A Simple and Rapid Method for the Removal of Unincorporated Nucleotides from Labeled Nucleic Acid", INTERNET CITATION, 28 December 1998 (1998-12-28), XP008130382, Retrieved from the Internet: URL:http: //www.roche-applied-science.com/P ROD_INF/ BIOCHEMI/No1_99/b199noet.h [retrieved on 2010-12-09]**
- **ROSENBLUM ET AL.: 'New dye-labeled terminators for improved DNA sequencing patterns' NUCLEIC ACIDS RESEARCH vol. 25, no. 22, 1997, pages 4500 - 4504, XP002909905**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 497 304 B1

## Description

## BACKGROUND OF THE INVENTION

With the advent of the Human Genome Project and the field of pharmacogenomics, which aims to correlate sequence polymorphisms with variations in drug responses and disease susceptibility, a heightened need for improved nucleic acid sequencing methods has become apparent.

The most commonly used sequencing methods are variants on the "Sanger" or "dideoxy" method, in which the enzymatic, template dependent incorporation of a chain-terminating dideoxynucleotide results in the generation of a collection of nucleic acid fragments each ending with the base carried by that analog. When a set of four such reactions is performed, one for each of the bases G, A, T and C, the electrophoretically-separated fragments will form a "ladder" from which the sequence can be read.

The efforts to map genomic sequence polymorphisms and mutations, particularly single nucleotide polymorphisms ("SNPs"), have spawned new sequencing technologies aimed at obtaining small amounts of sequence information (often single nucleotides) from a large number of nucleic acid samples. The so-called "minisequencing" methods are currently performed using fluorescently labeled dideoxynucleotides that are enzymatically incorporated opposite a SNP site.

Both "classical" sequencing methods and "minisequencing" methods are thus dependent upon chain terminating nucleotide analogs. The nucleotide terminators traditionally used in such methods are the dideoxy nucleotides, which are structurally similar to the "naturally occurring" deoxynucleotides but differ in the glycosyl component. The dideoxy chain terminating nucleotides contain a 2',3'-dideoxyribofuranosyl moiety.

Several dual-labeled nucleotide analogs are known in the art. For example, Rosenblum et al. (1997, Nucleic Acids Research, 25: 4500) teach the use of nucleotide analogs comprising a fluorescence resonance energy transfer (FRET) dye pair linked to the nucleobase. Incorporation of such analogs into a growing polynucletoide chain is detected by contacting the analog with light of a wavelength within the excitation spectrum of one of the dyes but not the other. The light emitted by the excited fluorophore then, in turn, excites the second dye, from which fluorescence emission is detected. Rosenblum et al. do not, however, teach linking a fluorescent moiety to the nucleobase of a nucleotide analog, and linking a quencher moiety to the terminal phosphate of the polyphosphate moiety of the same analog.

Williams (U.S. Pat. App. Pub. 20010018184) teaches a dual-labeled nucleotide analog in which a fluorophore is attached to the γ-phosphate of the polyphosphate moiety, and a quencher is linked elsewhere on the nucleotide analog, preferably linked to the 5' carbon of pyrimidine bases and to the 7' carbon of deazapurine bases. Upon incorporation of the analog taught by Williams into a growing polynucleotide chain, the phosphate group linked to the fluorescent moiety is cleaved off, thus separating the fluorescent moiety and the quencher, thereby permitting the fluorescent moiety to emit a detectable signal. Thus, incorporation of the analogs taught by Williams is detected by measuring fluorescently labeled pyrophosphate.

Because of the increasing demand for sequencing technologies dependent upon labeled chain terminators, and technologies which utilizes labeled nucleotides, there is a need in the art for alternative nucleotide analogs which may additionally function as chain terminators.

## SUMMARY OF THE INVENTION

The aspects and embodiments of the present invention are set forth in the accompanying claims.

The present disclosure provides a dual-labeled nucleotide comprising a fluorescent label and a quencher of that fluorescent label, wherein the quencher is attached to a phosphate moiety that is cleaved off of the nucleotide when the nucleotide is enzymatically incorporated into a polynucleotide. The fluorescent moiety is attached to the nucleotide at a position such that it is not cleaved off during the enzymatic incorporation of the nucleotide. The fluorescent moiety is located on the nucleotide at a distance from the quencher that results in at least a 2-fold quenching efficiency of the fluorescent moiety by the quencher moiety. The fluorescent moiety can be attached to the α-phosphate, the sugar moiety or the nucleobase.

In one aspect, the disclosure encompasses a nucleotide comprising a fluorescent label and a quencher of said fluorescent label, wherein said quencher is attached to a phosphate moiety that is cleaved off when said nucleotide is enzymatically incorporated into a polynucleotide.

In one embodiment, the fluorescent label is attached to the nucleobase of the nucleotide.

In another embodiment, the fluorescent label is attached to the sugar moiety of the nucleotide.

In another embodiment, the nucleotide is a nucleotide triphosphate. In a preferred embodiment, the quencher is attached to the γ phosphate of the triphosphate moiety of the nucleotide triphosphate.

In another embodiment, the nucleotide is a nucleotide tetraphosphate. In a preferred embodiment, the quencher is attached to the δ phosphate of the tetraphosphate moiety.

In another embodiment, the nucleotide comprises a sugar moiety selected from the group consisting of ribofuranosyl, 2'-deoxynibofuranosyl, 2', 3'-dideoxyribofuranosyl, phosphonomethoxyethyl, 2-oxyethoxymethyl, 2-hydroxymethoxymethyl, 3-pentenyl, oxetan, and pyran.

In anther embodiment, the quencher is a dark quencher.

In another embodiment, the quencher is fluorescent.

In another embodiment, the nucleotide comprises a nucleobase is selected from the group consisting of adenine, cytosine, guanine, thymine, uracil, 7-deazaguanine, 7-deazeedenosine, and hypoxanthine or an analog thereof.

In another embodiment, the fluorescent label is selected from the group consisting of R110, TAMRA, R6G and ROX.

In another aspect, the disclosure encompasses a dual-labeled nucleotide analog comprising a quencher moiety coupled to a polyphosphate moiety, coupled to a sugar moiety or an appropriate non-sugar linker, coupled to a nucleobase, which is, in turn, attached to a fluorescent moiety by a linker. This general structure is shown schematically below:

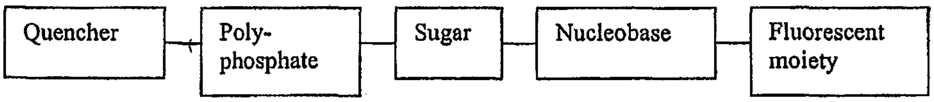


Thus, the invention relates to a dual-labeled nucleotide comprising a fluorescent label, a quencher of the fluorescent label, and a 5'-polyphosphate moiety wherein the quencher is attached to the polyphosphate moiety of the nucleotide and the fluorescent label is attached to the nucleobase of the nucleotide.

In another aspect, the quencher is attached to a phosphate moiety that is cleaved off when the nucleotide is enzymatically incorporated into a polynucleotide, and the fluorescent moiety is attached to the $\alpha$-phosphate, the sugar moiety, or the nucleobase of the nucleotide.

Dual-labeled nucleotides according to the disclosure are recognized by a nucleic acid polymerase and incorporated onto the 3' end of a growing oligo- or polynucleotide polymer by such polymerase.

In one embodiment, the polyphosphate moiety is selected from the group consisting of di, tri, or tetra-phosphate.

In another embodiment, the polyphosphate moiety is a triphosphate, and the quencher moiety is linked to the $\gamma$-phosphate of the triphosphate.

In a further embodiment, the sugar moiety is as defined herein, and can be, for example, ribofuranosyl (i.e., the "sugar moiety" of the ribonucleotides ATP, GTP, CTP and UTP found in mRNA *in vivo*), 2'-deoxyribofuranosyl (i.e., the "sugar moiety" of the deoxyribonucleotides dATP, dGTP, dCTP and dTTP found in DNA *in vivo*), 2', 3'-dideoxyribofuranosyl, phosphonomethoxyethyl, 2-oxyethoxymethyl, 2-hydroxymethoxymethyl, 3-pentenyl, oxetan, or pyran. Additional sugar moieties or non-sugar linker groups that substitute for the sugar moiety are described, for example, in Bioorg. Med. Chem. Lett. (1997) 7: 3013-3016, Nucl. Acids Res. (1999) 27: 1271-1274, and Nucleosides and Nucleotides (1993) 12: 83-93. Still others are depicted in Figure 2, which shows exemplary acyclic nucleoside analogs useful according to the invention. Each structure in Figure 2 includes adenine as the nucleobase, however, it should be understood that any nucleobase that permits polymerase enzyme recognition and incorporation and participates in base-pairing can be substituted.

In one embodiment, the quencher moiety is a dark quencher.

In another embodiment, the quencher is fluorescent.

In another embodiment, the nucleobase is as defined herein, and can be, for example, adenine, cytosine, guanine, thymine, uracil, hypoxanthine, 5-methylcytosine (5-me-C), 5-hydroxymethyl cytosine, xanthine, hypoxanthine, 2-aminoadenine, 6-methyl adenine, 6-methyl guanine, 2-propyl adenine, 2-propyl guanine, 2-thiouracil, 2-thiothymine, 2-thiocytosine, 5-halouracil, 5-halocytosine, 5-propynyl uracil, 5-propynyl cytosine, 6-azo uracil, 6-azo cytosine, 6-azo thymine, 5-uracil (pseudouracil), 4-thiouracil, 8-halo, 8-amino, 8-thiol, 8-thioalkyl, 8-hydroxyl adenine, 8-hydroxyl guanine, 5-halo uracil, 5-halo cytosine, 5-bromouracil, 5-bromocytosine, 5-trifluoromethyluracil, 5-trifluoromethylcytosine, 7-methylguanine, 7-methyladenine, 8-azaguanine, 8-azaadenine, 7-deazapurine, 7-deazaguanine, 7-deazaadenine, 3-deazaguanine, or 3-deazaadenine. Other modifications of the basic purine and pyrimidine ring structures that are accepted by nucleic acid polymerases are candidates for use as nucleobases. Such modified nucleobases and methods of obtaining them are known by those skilled in the art of designing modified base polymerase substrates. Nucleobase analogs useful according to the invention permit the nucleotide comprising such nucleobase to serve as a substrate for a nucleic acid polymerase.

In another embodiment, the fluorescent moiety is as defined herein and can be, for example, 4-acetamido-4'-isothiocyanatostilbene-2,2'-disulfonic acid, acridine, acridine isothiocyanate, 5-(2'-aminoethyl)aminonap- hthalene-1-sulfonic acid (EDANS), 4-amino-N-[3-vinylsulfonyl)phenyl]naphth- alimide-3,5 disulfonate, N-(4-anilino-1-naphthyl)maleimide; anthranilamide, BODIPY, Brilliant Yellow, coumarin, 7-amino-4-methylcoumarin (AMC, Coumarin 120),7-amino-4-trifluor- omethylcouluarin (Coumaran 151), cyanine dyes, cyanosine, 4',6-diaminidino-2-phenylindole (DAPI), 5',5"-dibromopyrogallol-sulfonaph- thalein (Bromopyrogallol Red), 7-diethylamino-3-(4'-isothiocyanatophenyl)-- 4-methylcoumarin, diethylenetriamine pentaacetate, 4,4'-diisothiocyanatodi- hydro-stilbene-2,2'-disulfonic acid, 4,4'-diisothiocyanatostilbene-2,2'-di- sulfonic acid, 5-[dimethylamino]naphthalene-1-sulfonyl chloride (DNS, dansylchloride), 4-dimethylaminophenylazophenyl-4'-isothiocyanate (DABITC), eosin, eosin isothiocyanate, erythrosin, erythrosin B, isothiocyanate, ethidium, fluorescein, 5-carboxyfluorescein (FAM),5-(4,6-dichlorotr- iazin-2-yl)aminofluorescein (DTAF), 2',7'-dimethoxy-4'5'-dichloro-6-carbox- yfluorescein (JOE), fluorescein, fluorescein isothiocyanate, QFITC, (XRITC), fluorescarnine, IR144, IR1446, Malachite Green isothiocyanate, 4-methylumbelliferoneortho cresolphthalein, nitrotyrosine, pararosaniline, Phenol Red, B-phycoerythrin, o-phthaldialdehyde, pyrene and derivatives: pyrene, pyrene butyrate, succinimidyl 1-pyrene, butyrate quantum dots, Reactive Red 4 (Cibacron.TM. Brilliant Red 3B-A) rhodamine, 6-carboxy-X-rhodamine (ROX), 6-carboxyrhodamine (R6G), lissamine rhodamine B sulfonyl chloride rhodamine (Rhod), R110, rhodamine B, rhodamine 123, rhodamine X isothiocyanate, sulforhodamine B, sulforhodamine 101, sulfonyl chloride derivative of sulforhodamine 101 (Texas Red), N,N,N',N'-tetramethyl-6-carboxyrhodamine (TAMRA), tetramethyl rhodamine, tetramethyl rhodamine isothiocyanate (TRITC), riboflavin, rosolic acid, Cy 3, Cy 5, Cy 5.5, Cy 7, IRD 700, IRD 800, La Jolla Blue, phthalo cyanine, Oregon green, or naphthalo cyanine.

In a preferred embodiment, a set of four nucleotides analogous to dG, dA, dT or dC is used, each labeled with a different fluorescent moiety. A set of such fluorescent moieties that can be used is, for example, R110, ROX, R6G, and TAMRA; each fluorescent moiety in the set fluoresces at a distinguishable wavelength, permitting simultaneous measurement of each nucleotide in the same sample.

In another embodiment, dA analogs are labeled with R110 or ROX, dG analogs are labeled with R110, dT analogs are labeled with ROX, and dC analogs are labeled with R110 or ROX.

The invention further encompasses a dual-labeled nucleotide analog having a general structure selected as follows:

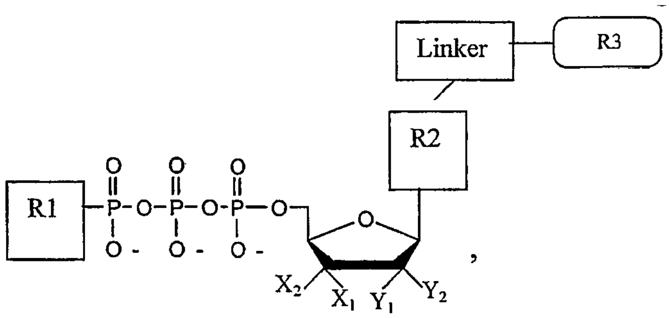

,

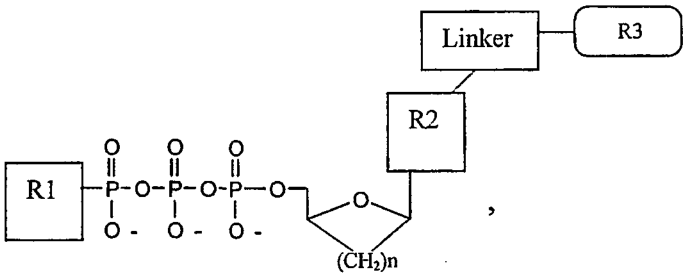

,

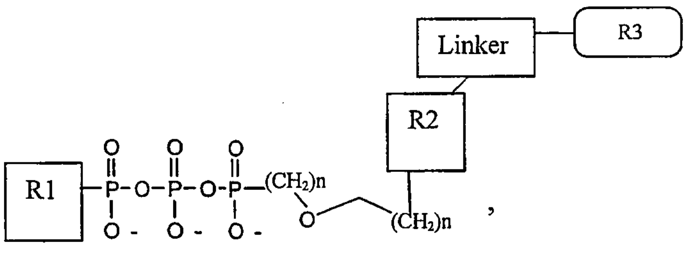

,

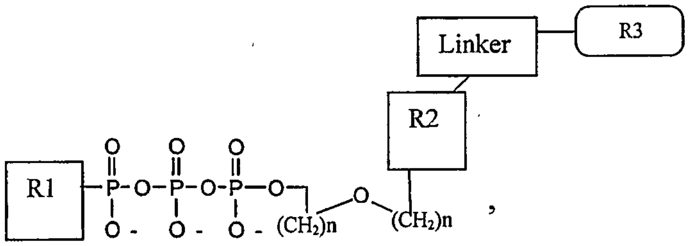

,

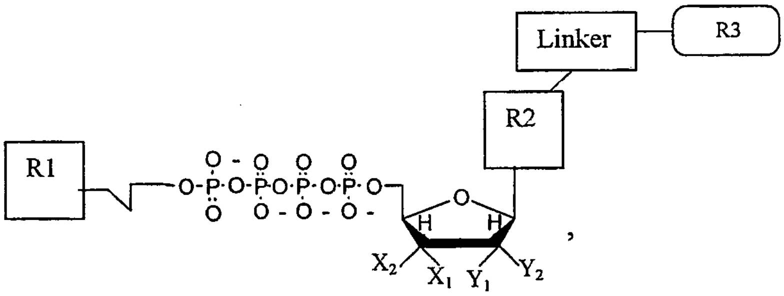

,

and

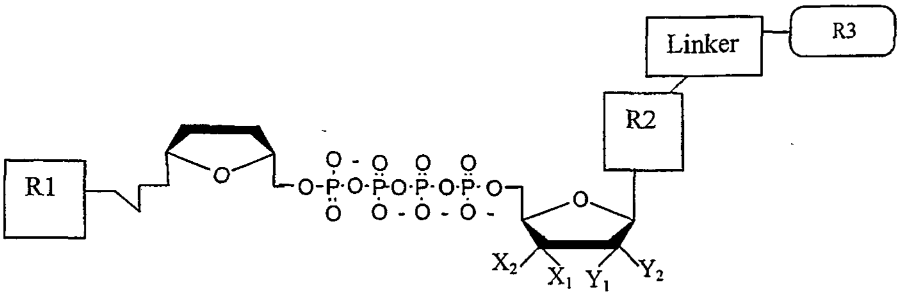

In this embodiment, R1 is a quencher moiety; R2 is a nucleobase; Linker is a moiety that covalently links R2 and R3; and R3 is a fluorescent moiety, the emission of which is quenched by the dark quencher. The nucleobase moiety R2 can be any nucleobase as the term is defined herein; examples of useful nucleobases are as described for nucleobases for the preceding embodiments. The fluorescent moiety R3 can be any fluorescent moiety as the term is defined herein, but must be quenched by the quencher moiety, R1. Examples of useful fluorescent moieties are as described for the preceding embodiments. In the above structures, $X_1$ $X_2$, $Y_1$ and $Y_2$ can each be H, OH, F, or $NH_2$ and n = 1 to 12.

In one embodiment, the quencher, R1 is a dark quencher.

In one embodiment, the linker is attached to the nucleobase at the N-4 or C-5 position when the nucleobase is a pyrimidine, or at the N-6, C-8, or C(N)-7 position when the nucleobase is a purine.

The invention further encompasses a method of synthesizing a polynucleotide, the method comprising contacting a nucleic acid polymerase enzyme with a a nucleotide comprising a fluorescent label and a quencher of that fluorescent label, wherein the quencher is attached to a phosphate moiety that is cleaved off when the nucleotide is enzymatically incorporated into a polynucleotide, under conditions permitting the extension of a nucleic acid primer annealed to a template nucleic acid, wherein the dual-labeled nucleotide analog is thereby incorporated into the nucleic acid primer. The process of enzymatic incorporation of the dual-labeled nucleotide involves the cleavage of the quencher from the dual-labeled nucleotide when the pyrophosphate is removed, such that the nucleotide retains only the fluorescent label moiety after incorporation. For the purposes of this specification, the dual-labeled nucleotides according to the invention are said herein to be "incorporated" even though it is acknowledged that the actual form incorporated is labeled only with a fluorescent moiety, due to the removal of the quencher moiety upon incorporation.

In one embodiment of the method, the step of contacting results in chain termination.

In a further embodiment of the method, the step of contacting permits the determination of nucleic acid sequence information about the template nucleic acid.

In one embodiment of the method, the polyphosphate moiety is selected from the group consisting of di, tri, or

tetra-phosphate.

In a further embodiment of the method, the polyphosphate moiety is a triphosphate, and the quencher moiety is linked to the γ-phosphate of the triphosphate.

In another embodiment of the method, the sugar moiety is as described in previous embodiments.

In another embodiment of the method, the quencher is a dark quencher.

In another embodiment of the method, the nucleobase is as defined herein and described above for the dual-labeleld nucleotide compositions of the invention.

In another embodimentof the method, the fluorescent moiety is as defined herein and described above for the dual-labeleld nucleotide compositions of the invention.

The present invention also provides a method of labeling a polynucleotide, the method comprising contacting a nucleic acid polymerase enzyme with a nucleotide comprising a fluorescent label and a quencher of said fluorescent label, wherein said quencher is attached to a phosphate moiety that is cleaved off when said nucleotide is enzymatically incorporated into a polynucleotide, under conditions permitting the extension of a nucleic acid primer annealed to a template nucleic acid, wherein the dual-labeled nucleotide analog is thereby incorporated into the nucleic acid primer. The process of enzymatic incorporation of the dual-labeled nucleotide involves the cleavage of the quencher from the dual-labeled nucleotide, such that the nucleotide retains only the fluorescent label moiety after incorporation.

In one embodiment of the method, the polyphosphate moiety is selected from the group consisting of di-, tri-, or tetra-phosphate.

In a further embodiment of the method, the polyphosphate moiety is a triphosphate, and the quencher moiety is linked to the γ-phosphate of the triphosphate.

In another embodiment of the method, the sugar moiety is as described above for the dual-labeled nucleotide compositions according to the invention.

In one embodiment of the method, the quencher moiety is a dark quencher. When the quencher is a dark quencher, probes labeled using dual-labeled nucleotides according to the invention have the advantage that they do not absolutely require purification to remove unincorporated label before use, because unincorporated label will not generate a signal. Only upon incorporation is the dark quencher cleaved off to generate a signal. This advantage allows the design of simplified assay formats that utilize enzymatic DNA synthesis (e.g., PCR, SDA, rolling circle amplification, etc.). Because unincorporated dual-labeled nucleotides demonstrate an attenuated fluorescent signal, the need for removal of unincorporated nucleotides is obviated, permitting the design of closed-tube assay formats (assay vessels need not be opened, thus reducing the level of contaminants added to the system).

In another embodiment of the method, the nucleobase is as described above for the dual-labeled nucleotide compositions according to the invention.

In another embodiment of the method, the fluorescent moiety is as described above for the dual-labeled nucleotide compositions according to the invention.

The invention further provides a method of determining sequence information about a template polynucleotide, the method comprising: annealing an oligonucleotide primer to a template polynucleotide to generate an annealed primer; contacting the annealed primer and the template with a nucleic acid polymerase enzyme in the presence of a nucleotide comprising a fluorescent label and a quencher of said fluorescent label, wherein said quencher is attached to a phosphate moiety that is cleaved off when said nucleotide is enzymatically incorporated into a polynucleotide, under conditions sufficient to permit the extension of the primer by the nucleic acid polymerase enzyme; and detecting the incorporation of the dual-labeled nucleotide analog into the primer, wherein the incorporation determines sequence information about the template polynucleotide.

In one embodiment of the method, the polyphosphate moiety is selected from the group consisting of di, tri, or tetra-phosphate. In a further embodiment, the polyphosphate moiety is a triphosphate, and the quencher moiety is linked to the γ-phosphate of the triphosphate.

In another embodiment of the method, the sugar moiety is as defined herein and described above for the dual-labeled nucleotide compositions according to the invention.

In another embodiment of the method, the quencher moiety is a dark quencher.

In another embodiment of the method, the nucleobase is as defined hererin and described above for the dual-labeled nucleotide compositions according to the invention.

In another further embodiment of the method, the fluorescent moiety is as defined hererin and described above for the dual-labeled nucleotide compositions according to the invention.

In one embodiment, the method of determining sequence information is performed on a solid support.

In another embodiment of the method, the incorporation of the dual-labeled nucleotide analog into the primer results in cleavage of the quencher moiety from the dual-labeled nucleotide analog.

In a further embodiment, the detection step comprises contacting the dual-labeled nucleotide analog with light of a wavelength that is within the excitation spectrum of the fluorescent moiety, and detecting the resulting emission of fluorescent light from the dual-labeled nucleotide analog.

Finally, the invention provides a kit comprising a dual-labeled nucleotide analog of the invention, wherein the kit further comprises, a nucleic acid polymerase, and an oligonucleotide primer. Kits of the invention can optionally comprise necessary reaction buffers and detailed instructions for performing the assay.

Definitions

As used herein, the term "dual-labeled nucleotide analog" refers to a nucleotide having the general structure:

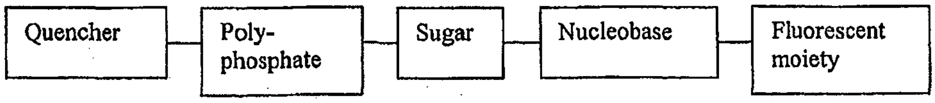


where the nucleobase represents a purine, 7-deazapurine or pyrimidine nucleobase or a nucleobase analog that permits Watson-Crick base pairing between the analog and the nucleobase of a nucleotide on an adjacent antiparallel oligo- or polynucleotide. The nucleobase moiety is preferably selected from the group consisting of adenine, cytosine, guanine, thymine, uracil and hypoxanthine, although modified forms and functional analogs of these are specifically contemplated (see below). The "polyphosphate group" is a di-, tri-, or tetraphosphate. The polyphosphate group is preferably a triphosphate having the structure:

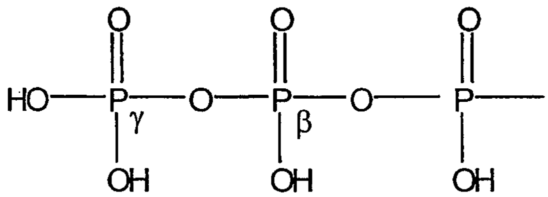

The phosphorus atom most distal to the sugar moiety of the nucleotide analog is referred to herein as the "gamma phosphorus." The dual-labeled nucleotide analog according to the invention will serve as a substrate for a nucleic acid polymerase enzyme to produce a nucleotide analog covalently attached to the 3' end of a nucleic acid primer annealed to a template nucleic acid strand. The dual-labeled nucleotide analog according to the invention will be incorporated opposite, and hydrogen bond with, a complementary nucleotide on the template strand. Further, a dual-labeled nucleotide analog according to the invention may act as a chain terminator for the template-directed polymerization of a polynucleotide by a nucleic acid polymerase.

In an alternative configuration of a "dual-labeled nucleotide analog" as the term is used herein, the fluorescent moiety is attached to the sugar moiety, rather than to the nucleobase moiety.

As used herein, the term "quencher moiety" refers to any fluorescence-modifying group that can attenuate at least partly (i.e., by at least 10%) the light emitted by a fluorescent group. This attenuation is referred to herein as "quenching". Hence, illumination of the "fluorescent moiety" of the dual-labeled nucleotide analog in the presence of the "quenching moiety" leads to an emission signal that is less intense than expected, or even completely absent. The quencher useful herein can itself be fluorescent, but absorb the energy emitted by the other fluorescent moiety on the dual-labeled nucleotide. In this instance, commonly referred to as Fluorescence Resonance Energy Transfer, or FRET, illumination of the "fluorescent moiety" attached to the nucleobase with light within the excitation spectrum of that moiety will result in non-radiative transfer from that fluorescent moiety to the "quencher moiety," which then emits at a different wavelength than the fluorescent moiety attached to the nucleobase. In this situation, the "quencher" emits light but, due to its attenuation of the emission of the "fluorescent moiety," is still considered a "quencher moiety." The activity of a given "quencher moiety" should always be considered relative to a given "fluorescent moiety." That is, a fluorescent molecule may itself be a quencher of the emission from one fluorescent molecule but not from another. As used herein, a "quencher" is a moiety that is added to a nucleotide or nucleotide analog, that is, the fluorescence quenching is not an intrinsic property of the nucleotide prior to the addition of a quencher moiety.

As used herein, a "dark quencher" refers to a quencher moiety that absorbs energy from an excited fluorophore, but which does not release fluorescent energy itself. "Dark quenchers" useful in the present invention include, but are not limited to 4-(dimethylamino)azobenzene (DABCYL) and its derivatives, dinitrophenyl, DABMI, malachite green, QSY 7, QSY 9, QSY 21, QSY 35 ("QSY" quenchers available from Molecular Probes, Inc., Eugene, OR, see U.S. Patent No. 6,329,205, incorporated herein by reference) and the black hole quenchers (BHQ) taught in WO01/86001.

As used herein, the term "phosphate moiety that is cleaved off when a nucleotide is enzymatically incorporated into a polynucleotide" refers to a phosphate group on a nucleoside, other than the $\alpha$-phosphate, which is retained as

part of the sugar-phosphate backbone of the polynucleotide upon incorporation. Those phosphate moieties cleaved off include, for example, the β, γ, and δ phosphates of a di-, tri- or tetraphosphate moiety.

As used herein, the phrase "distance from a quencher that results in at least a 2-fold quenching efficiency of a fluorescent moiety by the quencher moiety" refers to a distance, less than 100 Å, determined for a given fluorescent moiety:quencher pair through use of the formulae described herein for determining Förster's radius and quenching efficiency.

The term "oligonucleotide" as used herein includes linear oligomers of nucleotides or analogs thereof, including deoxyribonucleosides, ribonucleosides, and the like. Usually, oligonucleotides range in size from a few monomeric units, e.g. 3-4, to several hundreds of monomeric units. Whenever an oligonucleotide is represented by a sequence of letters, such as "ATGCCTG," it will be understood that the nucleotides are in 5'-3' order from left to right and that "A" denotes deoxyadenosine, "C" denotes deoxycytidine, "G" denotes deoxyguanosine, and "T" denotes thymidine, unless otherwise noted.

The term "nucleoside" as used herein refers to a compound consisting of a purine, deazapurine, or pyrimidine nucleoside base, e.g., adenine, guanine, cytosine, uracil, thymine, deazaadenine, deazaguanosine, and the like, linked to a pentose at the 1' position, including 2'-deoxy and 2'-hydroxyl forms, e.g. as described in Kornberg and Baker, DNA Replication, 2nd Ed. (Freeman, San Francisco, 1992). Nucleosides can also comprise "acyclo" sugar moieties, such as phosphonomethoxyethyl, 2-oxyethoxymethyl, 2-hydroxymethoxymethyl, etc., that permit recognition and incorporation by nucleic acid polymerase enzymes. Nucleosides also include, but are not limited to, synthetic nucleosides having modified base moieties and/or modified sugar moieties, e.g. those described herein or those described generally by Scheit, Nucleotide Analogs (John Wiley, N.Y., 1980). Suitable NTPs include both naturally occurring and synthetic nucleoside triphosphates, and include but are not limited to, ATP, dATP, ddATP, CTP, dCTP, ddCTP, GTP, dGTP, ddGTP, TTP, dTTP, ddTPP, UTP and dUTP. Preferably, the nucleoside triphosphates used in the methods of the present invention are selected from the group consisting of dATP, ddATP, dCTP, ddCTP, dGTP, ddGTP, dTTP, ddTTP, UTP and dUTP and mixtures thereof.

The term "nucleotide" as used herein refers to a phosphate ester of a nucleoside, e.g., mono, di, tri, and tetraphosphate esters, wherein the most common site of esterification is the hydroxyl group attached to the C-5 position of the pentose (or equivalent position of a non-pentose "sugar moiety").

The term "primer" refers to a linear oligonucleotide which specifically anneals to a unique polynucleotide sequence and allows for amplification of that unique polynucleotide sequence.

The phrase "sequence determination" or "determining a nucleotide sequence" in reference to polynucleotides includes determination of partial as well as full sequence information of the polynucleotide. That is, the term includes sequence comparisons, fingerprinting, and like levels of information about a target polynucleotide, or oligonucleotide, as well as the express identification and ordering of nucleosides, usually each nucleoside, in a target polynucleotide. The term also includes the determination of the identification, ordering, and locations of one, two, or three of the four types of nucleotides within a target polynucleotide.

The term "solid-support" refers to a material in the solid-phase that interacts with reagents in the liquid phase by heterogeneous reactions. Solid-supports can be derivatized with proteins such as enzymes, peptides, oligonucleotides and polynucleotides by covalent or noncovalent bonding through one or more attachment sites, thereby "immobilizing" the protein or nucleic acid to the solid-support.

As used herein, the term "complementary nucleotide" refers to a nucleotide in which, when conditions permit the annealing or hybridization of nucleic acid strands, the nucleobase of the nucleotide forms hydrogen bonds with the nucleobase of a given dual-labeled nucleotide analog of the present invention. The pattern of hydrogen bond formation between the respective complementary nucleobases will be as follows: adenine hydrogen bonding to thymine or uracil (two H bonds), guanine hydrogen bonding to cytosine (three H bonds), and hypoxanthine hydrogen bonding to adenine, cytosine or uracil (hypoxanthine is the nucleobase moiety of the ribonucleoside inosine).

As used herein, the term "nucleobase" refers to the heterocyclic nitrogenous base of a nucleotide or nucleotide analog. Nucleobases useful according to the invention include, but are not limited to adenine, cytosine, guanine, thymine, uracil, and hypoxanthine. Additional nucleobases that can be comprised by a dual-labeled nucleotide analog according to the invention include, but are not limited to naturally-occurring and synthetic derivatives of the preceding group, for example, pyrazolo[3,4-d] pyrimidines, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine, 3-deazaadenine, pyrazolo[3,4-d]pyrimidine, imidazo[1,5-a] 1,3,5 triazinones, 9-deazapurines, imidazo[4,5-d]pyrazines, thiazolo[4,5-d]pyrimidines, pyrazin-2-ones, 1,2,4- triazine, pyridazine; and 1,3,5 triazine. Nucleobases useful according to the invention will permit a nucleotide bearing that nucleobase

to be enzymatically incorporated into a polynucleotide chain and will form Watson-Crick base pairs with a nucleobase on an antiparallel nucleic acid strand.

As used herein, the phrase "Watson-Crick base pair" refers to a pair of hydrogen-bonded nucleobases on opposite antiparallel strands of nucleic acid. The well-known rules of base pairing first elaborated by Watson and Crick, require that adenine (A) pairs with thymine (T) or uracil (U), and guanine (G) pairs with cytosine (C), with the complementary strands anti-parallel to one another. The Watson-Crick pairing rules can be understood chemically in terms of the arrangement of hydrogen bonding groups on the heterocyclic bases of the oligonucleotide, groups that can either be hydrogen bond donors or acceptors. In the standard Watson-Crick geometry, a large purine base pairs with a small pyrimidine base; thus, the AT base pair is the same size as a GC base pair. This means that the rungs of the DNA ladder, formed from either AT or GC base pairs, all have the same length. Further recognition between bases is determined by hydrogen bonds between the bases. Hydrogen bond donors are heteroatoms (nitrogen or oxygen in the natural bases) bearing a hydrogen; hydrogen bond acceptors are heteroatoms (nitrogen or oxygen in the natural bases) with a lone pair of electrons. In the geometry of the standard Watson-Crick base pair, a six membered ring (in natural oligonucleotides, a pyrimidine) is juxtaposed to a ring system composed of a fused six membered ring and a five membered ring (in natural oligonucleotides, a purine), with a middle hydrogen bond linking two ring atoms, and hydrogen bonds on either side joining functional groups appended to each of the rings, with donor groups paired with acceptor groups.

As used herein, the term "Watson-Crick base pair" encompasses not only the standard AT, AU or GC base pairs, but also base pairs formed between nucleobases of nucleotide analogs comprising non-standard or modified nucleobases, wherein the arrangement of hydrogen bond donors and hydrogen bond acceptors permits hydrogen bonding between a non-standard nucleobase and a standard nucleobase or between two complementary non-standard nucleobase structures. One example of such non-standard Watson-Crick base pairing is the base pairing engaged in by the nucleotide analog inosine, wherein the hypoxanthine nucleobase forms two hydrogen bonds with adenine, cytosine or uracil.

As used herein, the phrase "nucleobase analog capable of forming Watson-Crick base pairs with a nucleobase on an adjacent antiparallel nucleic acid strand" refers to a nucleobase other than one of adenine, cytosine, guanine, thymine and uracil, that, when incorporated into an oligo- or polynucleotide strand has hydrogen bond donors or acceptors located such that the nucleobase can form hydrogen bonds with hydrogen bond acceptors or donors, respectively, present on a nucleobase or nucleobase analog on an adjacent antiparallel nucleic acid strand. U. S. Patent No. 6,001,983, which is incorporated herein by reference, provides guidance on the design of nucleobase analogs capable of forming non-standard Watson-Crick base pairs, and methods of analyzing their base pair interactions. A "nucleobase analog capable of forming Watson-Crick base pairs with a nucleobase on an adjacent antiparallel nucleic acid strand" useful according to the invention will permit the template-dependent enzymatic incorporation of a nucleotide analog comprising such a nucleobase analog into a polynucleotide chain.

As used herein, the term "linker" refers to the chemical group or groups that join a fluorescent moiety to a nucleotide analog, or which can join a quencher moiety to a phosphate group. The dual-labeled nucleotide analog can be generated by reacting a nucleotide, modified on the nucleobase to contain a reactive group (e.g., an amine on an allyl amine or alkynyl amine), with a fluorescent dye bearing a complementary reactive group (e.g., a succinimidyl (NHS) group). Alternatively, the nucleotide analog, modified to contain a reactive group on the nucleobase, can be reacted first with an intermediate linker moiety, such as an ethylene oxy group or a commercially available heterobifunctional reagent, and then reacted with a fluorescent dye with an appropriate reactive group (e.g., an NHS group). In either instance, the "linker" according to the invention is considered to be the chemical entity or entities between the nucleobase and the fluorescent dye. That is, the "linker" encompasses any modifying group added to the nucleobase in order to provide a reactive group for the attachment of a dye or an intermediate linking group, and any such intermediate linking group. The term "linker" alternatively refers to the chemical entity or "spacer" unit connecting the quencher to the gamma phosphate. Structures for this "linker," also referred to herein as "Linker 2" or "L2," are described herein below.

As used herein, the phrase "differentially labeled" means that one entity is labeled with a first detectable moiety and another entity is labeled with a second detectable moiety, and that the signals from the first and second detectable moieties can be distinguished. A "distinguishable fluorescent label" refers to a fluorescent label in which the emission peak can be distinguished from another fluorescent label present in the same mixture; generally, if the peak emission wavelengths of two fluorophores differ by at least 20 nm, they are considered to be distinguishable fluorophores.

As used herein, the term "chain terminator" refers to a nucleotide analog that serves as a substrate for a nucleic acid polymerase enzyme, but once incorporated onto the end of a growing polynucleotide chain, the analog cannot itself serve as a substrate for the attachment of subsequent nucleotide residues. A chain terminator lacks the hydroxyl group on the sugar moiety necessary for the terminator to serve as a substrate for subsequent enzymatic nucleotide addition. Classic examples of chain terminators include the dideoxynucleoside triphosphates ddA, ddC, ddG, and ddT, although other analogs can also serve as chain terminators (e.g., the phosphonomethoxyethyl nucleotide analogs).

As used herein, the phrase "determining sequence information" refers to the process wherein at least one nucleotide in a polynucleotide sequence is identified. Thus, the phrase "determining sequence information" encompasses

both "classical" chain terminator sequencing ("Sanger method," Sanger et al., 1975, J. Mol. Biol., 94:441), which can provide the sequences of hundreds to thousands of contiguous nucleotides in a single set of reactions, as well as the so-called "minisequencing" methods useful for identifying, for example, single base differences in a sequence relative to a standard.

As used herein, the phrase "nucleic acid polymerase enzyme" refers an enzyme that catalyzes the template-dependent polymerization of nucleoside triphosphates to form primer extension products that are complementary to one of the nucleic acid strands of the template nucleic acid sequence. A nucleic acid polymerase enzyme initiates synthesis at the 3' end of an annealed primer and proceeds in the direction toward the 5' end of the template. Numerous nucleic acid polymerases are known in the art and commercially available. Preferred nucleic acid polymerases are thermostable, i.e., they retain function after being subjected to temperatures sufficient to denature annealed strands of complementary nucleic acids.

As used herein, the phrase "conditions permitting the extension of a nucleic acid primer annealed to a template nucleic acid" refers to those conditions of salt concentration (metallic and non-metallic salts), pH, temperature, and necessary cofactor concentration under which a given polymerase enzyme catalyzes the extension of an annealed primer. Conditions for the primer extension activity of a wide range of polymerase enzymes are known in the art. As one example, conditions permitting the extension of a nucleic acid primer by Taq polymerase include the following (for any given enzyme, there can and often will be more than one set of such conditions): reactions are conducted in a buffer containing 50 mM KCl, 10 mM Tris (pH 8.3), 4 mM $MgCl_2$, (200 $\mu$M of one or more dNTPs and/or a chain terminator may be included, depending upon the type of primer extension or sequencing being performed); reactions are performed at 72°C.

As used herein, the term "fluorescent moiety" refers to a chemical group that absorbs light energy of one wavelength and, upon such absorbtion, is excited to emit light energy at another wavelength. "Fluorescent moieties" useful in the present invention include, but are not limited to 4-acetamido-4'-isothiocyanatostilbene-2,2'-disulfonic acid, acridine, acridine isothiocyanate, 5-(2'-aminoethyl)aminonap- hthalene-1-sulfonic acid (EDANS), 4-amino-N-[3-vinylsulfonyl)phenyl]naphth- alimide-3,5 disulfonate, N-(4-anilino-1-naphthyl)maleimide; anthranilamide, BODIPY, Brilliant Yellow, coumarin, 7-amino-4-methylcoumarin (AMC, Coumarin 120),7-amino-4-trifluor- omethylcouluarin (Coumaran 151), cyanine dyes, cyanosine, 4',6-diaminidino-2-phenylindole (DAPI), 5',5"-dibromopyrogallol-sulfonaph- thalein (Bromopyrogallol Red), 7-diethylamino-3-(4'-isothiocyanatophenyl)-- 4-methylcoumarin, diethylenetriamine pentaacetate, 4,4'-diisothiocyanatodi- hydro-stilbene-2,2'-disulfonic acid, 4,4'-diisothiocyanatostilbene-2,2'-di- sulfonic acid, 5-[dimethylamino]naphthalene-1-sulfonyl chloride (DNS, dansylchloride), 4-dimethylaminophenylazophenyl-4'-isothiocyanate (DABITC), eosin, eosin isothiocyanate, erythrosin, erythrosin B, isothiocyanate, ethidium, fluorescein, 5-carboxyfluorescein (FAM),5-(4,6-dichlorotr- iazin-2-yl)aminofluorescein (DTAF), 2',7'-dimethoxy-4'5'-dichloro-6-carbox- yfluorescein (JOE), fluorescein, fluorescein isothiocyanate, QFITC, (XRITC), fluorescamine, IR144, IR1446, Malachite Green isothiocyanate, 4-methylumbelliferoneortho cresolphthalein, nitrotyrosine, pararosaniline, Phenol Red, B-phycoerythrin, o-phthaldialdehyde, pyrene and derivatives: pyrene, pyrene butyrate, succinimidyl 1-pyrene, butyrate quantum dots, Reactive Red 4 (Cibacron.TM. Brilliant Red 3B-A) rhodamine, 6-carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'-tetramethyl-6-carboxyrhodamine (TAMRA), tetramethyl rhodamine, tetramethyl rhodamine isothiocyanate (TRITC), riboflavin, rosolic acid, Cy 3, Cy 5, Cy 5.5, Cy 7, IRD 700, IRD 800, La Jolla Blue, phthalo cyanine, Oregon green, and naphthalo cyanine. As used herein, a "fluorescent moiety" is added to a nucleotide or nucleotide analog, i.e., the fluorescence is not an intrinsic property of the nucleotide or nucleotide analog prior to the addition of a fluorescent moiety.

It is often useful to use a set of spectrally distinguishable fluorescent moieties that emit fluorescent energy at wavelengths that can be distinguished by fluorescent detection equipment (for example, the ABI Prism™ 377 Sequencer) when two or more such dyes are present in one sample. An example of a set of spectrally distinguishable fluorescent dyes useful for nucleotide sequencing reactions is rhodamine-6G (R6G), rhodamine 110 (R110), tetramethyl rhodamine (TAMRA) and rhodamine X (ROX) (available from Molecular Probes, Eugene, OR). Another set of other spectrally distinguishable fluorescent dyes useful for nucleic acid sequencing reactions include, for example, the variants dichloro-R6G, dichloro-ROX, dichloro-R110 and dichloro-TAMRA (Applied Biosystems, Inc., Foster City, CA).

As used herein, a "sugar moiety" refers to a moiety which occupies a position in the dual-labeled nucleotide analog relative to the other components of the dual-labeled nucleotide analog which is equivalent to the position occupied by the ribofuranose sugar ring in a traditional nucleotide. A "sugar moiety" as used herein may be a furanose or pyranose sugar ring comprising a hydroxyl group at both the 2' and 3' carbons, or wherein one or both of the hydroxyl groups bonded to the 2' and 3' carbons is replaced with -H. A "sugar moiety" as used herein also refers to a non-pyrofuranose sugar ring including, but not limited to the following structures:

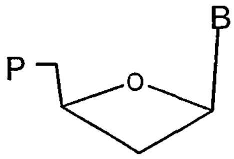
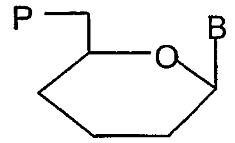
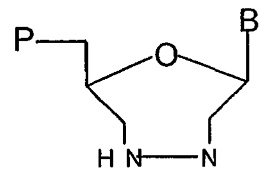

wherein B is a nucleobase linked to a fluorescent moiety, and wherein P is a polyphosphate moiety. Alternatively, a "sugar moiety" as used herein may refer to an acyclic group which occupies the same position in the dual-labeled nucleotide analog as the pyrofuranose sugar ring in a traditional nucleotide, provided that the dual-labeled nucleotide analog comprising the acyclic sugar moiety is capable of being incorporated into a polynucleotide chain in a manner similar to that of a nucleotide which contains a pyrofuranose sugar ring. Such incorporation is preferably enzymatic. Such acyclic moieties include, but are not limited to the following structures:

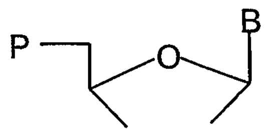
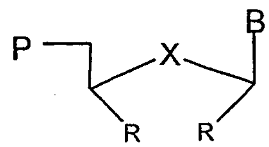

wherein B is a nucleobase linked to a fluorescent moiety, P is a polyphosphate moiety, X is $CH_2$ or $CF_2$, and R is $CH_3$, $CH_2$-R, R=OH, SH, halogen, $NH_2$, etc. or $(CH_2)_nCH_2$-R, n=0-6, R=OH, SH, halogen, $NH_2$, etc. The incorporation of a nucleotide bearing an alternative group in place of the standard sugar moiety will often be incorporated and/or terminate polymerization with greater or lesser efficiency than the standard nucleotides; where desired, polymerase enzymes can be tailored according to methods known in the art in order to improve the incorporation/termination efficiency with respect to a given alternative nucleotide structure.

## BRIEF DESCRIPTION OF THE FIGURES

Figure 1 is a schematic diagram showing several embodiments of the dual-labeled nucleotide analogs of the present invention.

Figure 2 shows the structures of several exemplary acyclic nucleoside analogs useful for the generation of dual labeled nucleotides according to the invention.

Figure 3 shows examples of dual labeled NTPs according to one embodiment of the invention with dyes attached to the "sugar" moiety.

Figure 4 shows examples of dual labeled NTPs according to one embodiment of the invention with dyes attached to the $\alpha$-phosphate moiety.

Figure 5 shows the structure of one embodiment of a dual labeled nucleotide according to the invention, 7-deaza-guanosine.

## DETAILED DESCRIPTION

<u>Dual-labeled Nucleotide Analogs</u>

The present disclosure provides a dual-labeled nucleotide analog comprising a quencher moiety coupled to a polyphosphate moiety, coupled to a sugar moiety, coupled to a nucleobase, which is, in turn, attached to a fluorescent moiety by a linker. The dual-labeled nucleotides of the present disclosure have the general structure:

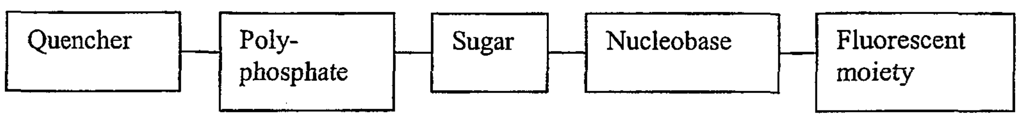

where the nucleobase represents a purine, 7-deazapurine or pyrimidine nucleobase or a nucleobase analog that permit Watson-Crick base pairing between the analog and the nucleobase of a nucleotide on an adjacent antiparallel oligo- or polynucleotide. The nucleobase moiety is preferably selected from the group consisting of adenine, cytosine, guanine, thymine, uracil and hypoxanthine, although modified forms and functional analogs of these are specifically contemplated (see below).

*Quenchers*

The quencher moiety can be a fluorescence-modifying group that can attenuate at least partly the light emitted by a fluorescent moiety, referred to as quenching. Hence, illumination of the fluorescent moiety of the dual-labeled nucleotide analog in the presence of the quenching moiety leads to an emission signal that is less intense than expected, or even completely absent. The quenching moiety is be a moiety that absorbs energy from an excited fluorophore, but which does not release fluorescent energy itself. Such a quenching moiety is referred to as a dark quencher. Dark quenchers useful in the present invention include, but are not limited to 4-(dimethylamino)azobenzene (DABCYL) and its derivatives, dinitrophenyl, DABMI, malachite green, QSY 7, QSY 9, QSY 21, QSY 35 ("QSY" quenchers available from Molecular Probes, Inc., Eugene, OR; see U.S patent No. 6,329,205) and the black hole quenchers (BHQ) taught in WO01/86001.

There is a great deal of practical guidance available in the literature for providing an exhaustive list of fluorescent and dark quenchers (also referred to as chromogenic molecules) and their relevant optical properties (see, for example, Berlnan, Handbook of Fluorescence Spectra of Aromatic Molecules, 2nd Edition (Academic Press, New York, 1971); Griffiths, Colour and Constitution of Organic Molecules (Academic Press, New York, 1976); Bishop, Ed., Indicators (Pergamon Press, Oxford, 1972); Haugland, Handbook of Fluorescent Probes and Research Chemicals (Molecular Probes, Eugene, 1992) Pringsheim, Fluorescence and Phosphorescence (Interscience Publishers, New York, 1949); and the like. Further, there is extensive guidance in the literature for derivatizing fluorophore and quencher molecules for covalent attachment via common reactive groups that can be added to a nucleotide, as exemplified by the following references: Haugland (supra); Ullman et al., U.S. Pat. No. 3,996,345; and Khanna et al., U.S. Pat. No. 4,351,760.

Quenching efficiency as measured in any particular experiment depends on the purity of the dye-quencher pair (contaminating free dye or cleaved molecules fluoresce); the electronic coupling and physical distance between dye and quencher (closer is usually better); and the excited-state lifetime of the dye (the longer the time, the greater the chances for electron transfer). Quenching efficiency increases as the overlap between dye emission and quencher absorption spectra increases.

While the exact distancees vary with the identity of the dye/quencher pair, efficient quenching generally requires dye and quencher to be less than 100 Å apart, preferably 10-100 Å apart, more preferably 20-80 Å apart, and more preferably 30-50 Å apart. The distance between donor/acceptor or donor/quencher pairs at which energy transfer is 50% efficient (i.e., 50% of activated donors are deactivated by FRET) is referred to as the Förster radius ($R_o$). The Förster radius is calcualted according to the formula:

$$R_o = [8.8 \times 10^{23} \times \kappa^2 \times n^{-4} \times QY_D \times J(\lambda)]^{1/6} \text{ Å},$$

in which: $\kappa^2$ is the dipole orientation factor (ranges from 0 to 4; k2 = 2/3 for randomly oriented donors and acceptors); $QY_D$ is the fluorescence quantum yield of the donor in the absence of the acceptor (quantum yield can be determined by one of skill in the art); n is the refractive index; $J(\lambda)$ is the spectral overlap integral, which is calculated by the formula:

$$J(\lambda) = \int \varepsilon_A(\lambda) \times F_D(\lambda) \times \lambda^4 \, d\lambda \text{ cm}^3/\text{M}$$

in which $\varepsilon_A$ is the extinction coefficient of the acceptor/quencher and $F_D$ is the fluorescence emission intensity of the donor as a fraction of the total integrated intensity.

In one embodiment, the present disclosure provides nucleotide analog molecules having a $\gamma$-phosphate with a fluorophore moiety attached thereto. The fluorophore moiety exists quenched with at least about a 2 fold quenching efficiency, preferably at least about a 5 fold quenching efficiency when the $\gamma$-phosphate is attached to the nucleotide analog and is unquenched i.e., is fluorescent, when the $\gamma$-phosphate is detached from the NTP. Preferably, the fluorophore moiety exists quenched with at least about a 3 fold quenching efficiency to about 100 fold quenching efficiency. In a more preferred embodiment, the fluorophore moiety exists quenched with at least about a 100 fold quenching efficiency to about a 1000 fold quenching efficiency.

The quenching efficiency of the NTPs of the present invention is a routine parameter easily determined. As will

be apparent to those of skill in the art, quenching efficiency can be measured in a fluorometer optionally having laser excitation. The quenching efficiency is equal to

$$F_o\text{-}F/F_o$$

wherein $F_o$ is fluorescence of the nucleotide analog without quenching and F is the quenched fluorescence. Because there is no certain way to eliminate all fluorescence in a sample of quenched nucleotide analog, the unquenched measurements, $F_o$, can be taken in a separate sample containing dye alone and the quenched measurements, F, can be made on the same concentration of quenched nucleotide analog. In a dual-labeled nucleotide according to the invention, the quenching efficiency is influenced by the location of the fluorophore relative to the quencher located on a phosphate that is cleaved off when the nucleotide is enzymatically incorporated into a polynucleotide. The decision as to where to locate the fluorophore relative to the quencher can take into consideration the Förster's radius of the pair. The determination of whether the quenching efficiency is at least two-fold when the fluorophore is at a particular location on the molecule can be made by one skilled in the art using standard methods and formulae provided herein or known in the art.

The nucleotide compounds of the present invention have at least 2 fold quenching efficiency prior to enzymatic incorporation which separates the quencher from the fluorophore. A fully fluorescent dye has a $F_o$ value of 1, whereas a dye quenched by 90% has an F value of 0.100. A compound quenched by 90%, has a quenching efficiency of 0.9 or is 10 fold quenched. Preferably the quenching efficiency of a compound of the present invention is about at least 2 fold to about 1000 fold, preferably the quenching efficiency of a compound of the present invention is about at least 5 fold to about 1000 fold, and more preferably, the quenching efficiency is about at least 10 fold to about 1000 fold.

In the present invention, detection of the dual-labeled nucleotide analog depends on generating a fluorescent signal by dequenching, or turning on, a quenched fluorescent dye in response to the liberation of pyrophosphate from the dual-labeled nucleotide analog. Given the location of the quencher moiety, that is, coupled to the distal phosphate in the polyphosphate moiety, cleavage of the polyphosphate moiety upon incorporation of the analog in a growing polynucleotide chain results in pyrophosphate/quencher moiety liberation and dequenching of the fluorescent moiety linked to the nucleobase. Efficient quenching provides a lower background fluorescence, enhancing the signal-to-noise ratio upon dequenching by pyrophosphate. Incomplete quenching results in a low level fluorescence background from each dye molecule. Additional background fluorescence is contributed by a few of the dye molecules that are fully fluorescent because of accidental (i.e., pyrophosphate-independent) dequenching, for example by breakage of a bond connecting the dye to the quencher moiety. Thus, the background fluorescence has two components: a low-level fluorescence from all dye molecules, referred to herein as "distributed fluorescence background" and full-strength fluorescence from a few molecules, referred to herein as "localized fluorescence background".

The quencher moiety can be either derivatized for attachment to the γ-phosphate (or distal phosphate in the case of a tetraphosphate) directly, or may be attached via a linker as described below for the fluorescent moiety attachment to the nucleobase, using techniques which are well known to those of skill in the art (see for example, U.S. Pub. App. 20010018184)

*Polyphosphate*

The polyphosphate group is a di-, tri-, or tetra-phosphate. The polyphosphate group is preferably a tri-phosphate having the structure:

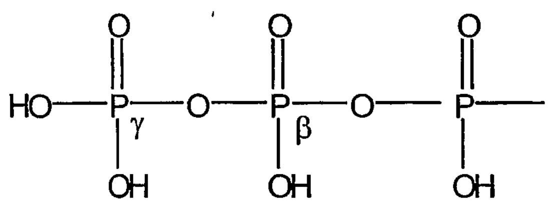

The phosphorus atom most distal to the sugar moiety of the nucleotide analog is referred to herein as the "gamma phosphorus." The dual-labeled nucleotide analog according to the invention will serve as a substrate for a nucleic acid polymerase enzyme to produce a nucleotide analog covalently attached to the 3' end of a nucleic acid primer annealed to a template nucleic acid strand. The dual-labeled nucleotide analog according to the invention will be incorporated opposite, and hydrogen bond with, a complementary nucleotide on the template strand. Further, a dual-labeled nucleotide analog according to the invention may act as a chain terminator for the template-directed polymerization of

a polynucleotide by a nucleic acid polymerase provided that the sugar moiety is a 2', 3'-dideoxy sugar moiety, or lacks hydroxyl groups in positions equivalent to the 2' and 3' carbons of a pyrofuranose sugar. Alternatively, the polyphosphate may be a tetraphosphate, in which the quencher moiety is coupled to the phosphate molecule which is most distal from the sugar moiety.

*Sugar Moiety*

The sugar moiety is a moiety which occupies a position in the dual-labeled nucleotide analog relative to the other components of the dual-labeled nucleotide analog which is equivalent to the position occupied by the pyrofuranose sugar ring in a traditional nucleotide. A sugar moiety may be a pyrofuranose sugar ring comprising a hydroxyl group at both the 2' and 3' carbons, or wherein one or both of the hydroxyl groups bonded to the 2' and 3' carbons is replaced with -H. A "sugar moiety" as used herein also refers to a non-pyrofuranose sugar ring including, but not limited to the following structures:

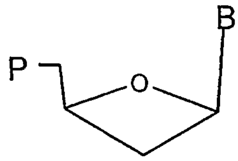
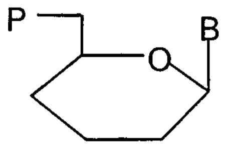
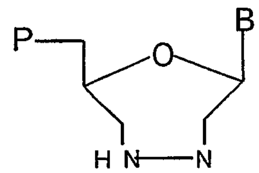

wherein B is a nucleobase linked to a fluorescent moiety, and wherein P is a polyphosphate moiety. Alternatively, a sugar moiety may be an acyclic group which occupies the same position in the dual-labeled nucleotide analog as the pyrofuranose sugar ring in a traditional nucleotide, provided that the dual-labeled nucleotide analog comprising the acyclic sugar moiety is capable of being incorporated into a polynucleotide chain in a manner similar to that of a nucleotide which contains a pyrofuranose sugar ring. Such acyclic moieties include, but are not limited to the following structures:

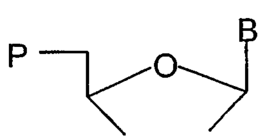
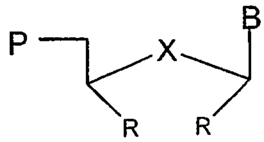

wherein B is a nucleobase, P is a polyphosphate moiety, X is $CH_2$ or CF, and each occurrence of R is $CH_3$, CH, or CF.

*Nucleobase*

The nucleobase is a heterocyclic nitrogenous base of a nucleotide or nucleotide analog. Nucleobases useful according to the invention include, but are not limited to adenine, cytosine, guanine, thymine, uracil, and hypoxanthine. Additional nucleobases that can be comprised by a dual-labeled nucleotide analog according to the invention include, but are not limited to naturally-occurring and synthetic derivatives of the preceding group, for example, pyrazolo[3,4-d] pyrimidines, 5-methylcytosine (5-me-C), 5-hydroxymethyl cytosine, xanthine, hypoxanthine, 2-aminoadenine, 6-methyl and other alkyl derivatives of adenine and guanine, 2-propyl and other alkyl derivatives of adenine and guanine, 2-thiouracil, 2-thiothymine and 2-thiocytosine, 5-halouracil and cytosine, 5-propynyl uracil and cytosine, 6-azo uracil, cytosine and thymine, 5-uracil (pseudouracil), 4-thiouracil, 8-halo, 8-amino, 8-thiol, 8-thioalkyl, 8-hydroxyl and other 8-substituted adenines and guanines, 5-halo particularly 5-bromo, 5-trifluoromethyl and other 5-substituted uracils and cytosines, 7-methylguanine and 7-methyladenine, 8-azaguanine and 8-azaadenine, 7-deazaguanine and 7-deazaadenine and 3-deazaguanine, 3-deazaadenine, pyrazolo[3,4-d]pyrimidine, imidazo[1,5-a]1,3,5 triazinones, 9-deazapurines, imidazo[4,5-d]pyrazines, thiazolo[4,5-d]pyrimidines, pyrazin-2-ones, 1,2,4- triazine, pyridazine; and 1,3,5 triazine. Nucleobases useful according to the invention will permit a nucleotide bearing that nucleobase to be enzymatically incorporated into a polynucleotide chain and will form Watson-Crick base pairs with a nucleobase on an antiparallel nucleic acid strand.

*Linker Moiety*

The dual-labeled nucleotide analogs of the present invention comprise a quencher moiety attached to the most distal phosphate of the polyphosphate moiety, and a fluorescent moiety linked via a linker, or linker arm to the nucleobase moiety. The linker arm can be attached to the nucleobase at any position that does not interfere with the ability of the

nucleobase to participate in Watson-Crick base pairing. For example, linker arm attachment at the N-4 or C-5 position of pyrimidines is acceptable. An alternative structure for a pyrimidine analog, has the linker arm attached at N-1(3), which is spatially equivalent to C-5. The linker arm can be attached to purines at either N-6, C-8 or C(N)7. When an alternative ring system is chosen (such as pyrazolo[3,4-d]pyrimidine) the linker should be positioned to be structurally equivalent to the acceptable positions on a purine nucleotide. In one embodiment, a dual-labeled nucleotide analog of the present invention also comprises a linker connecting a quencher moiety to the y-phosphate group as described below.

One common way to add a fluorescent label to a target molecule is to react an NHS ester of the dye with a reactive amino group on the target. Nucleotides can be modified to carry a reactive amino group by, for example, inclusion of an allyl amine group on the nucleobase. Labeling via allyl amine is described, for example, in U.S. Patent Nos. 5,476,928 and 5,958,691, which are incorporated herein by reference. While any nucleotide can be allyl amine modified, dUTP, dGTP and dTTP are perhaps best suited for situations, such as sequencing, in which maintenance of the natural hydrogen bonding capacity is called for. dUTP is modified by placing the aminoallyl group on the C5 position of the nucleobase. This position does not participate in hydrogen bonding necessary for nucleic acid heteroduplex formation. In contrast, dATP and dCTP are generally modified at the C6 position and the C4 position of the nucleobases, respectively. Generally, the alkylamino group is positioned on 7-deazapurine nucleotides (7-deazaadenosine, 7-deazaguanosine) at the 7-position. These sites do participate in hydrogen bonding in the heteroduplex, which makes them less attractive as sites for linker-mediated labeling.

A variety of linkers are useful for joining a detectable moiety (e.g., a fluorescent dye) to a nucleotide analog useful according to the invention. As used herein, the term "linker" refers to the chemical group or groups that join a fluorescent moiety, to a nucleotide analog. The labeled nucleotide analog can be generated by reacting a nucleotide analog, modified on the nucleobase to contain a reactive group (e.g., an amine on an allyl amine or alkynyl amine), with a fluorescent dye bearing a complementary reactive group (e.g., a succinimidyl (NHS) group). Alternatively, the nucleotide, modified to contain a reactive group on the nucleobase, can be reacted first with an intermediate linker moiety, such as an ethylene oxy group, and then reacted with a fluorescent dye with an appropriate reactive group (e.g., an NHS group). In either instance, the "linker" according to the invention is considered to be the chemical entity or entities between the nucleobase and the fluorescent dye. That is, the "linker" encompasses any modifying group added to the nucleobase in order to provide a reactive group for the attachment of a label or an intermediate linking group, and any such intermediate linking group.

Linkers can comprise, for example, an alkyl, allyl, or alkynyl amine modifying group attached to the nucleobase. As an alternative, linkers can comprise one or more ethylene oxy moieties. The linkers also include any chemical linking moieties located between the fluorescent dye and the reactive functionality (e.g., NHS group) attached to the dye before reaction with the nucleobase or intermediate linker.

The following figures depict the general chemical structure of linkers useful according to the invention. The figures and examples are meant to be exemplary; many additional structures known to those skilled in the art can serve the equivalent purpose of attaching a detectable marker, e.g., a fluorophore, to a nucleotide analog according to the invention.

Schematic structures for adenine and cytosine analogs useful according to the invention are shown below:

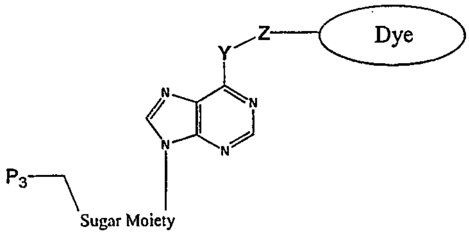

"A"

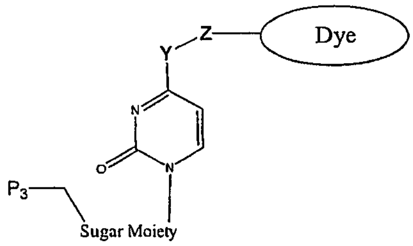

"C"

The linker "Y" on the A and C analogs can be described as follows. The most common linkers are essentially diamines connected to a carboxyl group on the dye, effecting an amide linkage. However, linkers can alternatively comprise S, O, or C reactive group termini, rather than N reactive group termini, on either or both ends of the linker moiety. The Z moiety is selected to be reactive with the group at the terminus of the linker that is not attached to the nucleobase. Thus, for example, when the terminus of linker Y that is not attached to the nucleobase is an amine, Z can represent a carboxyl moiety on the dye (or Z represents an additional linker plus a carboxyl moiety). Z can thus also be an amine, O, or S reactive group, as long as it is reactive with the free terminus of the selected linker Y.

Y can be selected, for example, from: hexanediamine, 4,7,10- trioxatriundecane-1,13-diamine, etc. or PEG-4-diamine. Alternatively, Y can be selected for example, from: $-NH-(CH_2)_3-O-(CH_2)_2-O-(CH_2)_2-O-(CH_2)_3-NH-$;

$-NH-(CH_2)_n-NH-$, where n=2-8; $-NH-(CH_2)_2-O-(CH_2)_2-O-(CH_2)_2-O-(CH_2)_2-O(CH_2)_2-NH-$; and $-NH-[(CH_2)_2-O]_n-NH-$, where n = 2-6.

Alternatively, the linker can have a terminal carboxyl (and be attached to an amino group, Z, on the dye). Thus, linkers can be selected, for example, from: $-NH-(CH_2)_3-O-(CH_2)_2-O-(CH_2)_2-O-(CH_2)_3-C(O)-$; $-NH-(CH_2)_n-$ C(O)-, where n=2-8; $-NH-(CH_2)_2-O-(CH_2)_2-O-(CH_2)_2-O-(CH_2)_2-O(CH_2)_2C(O)-$; and $-NH-[(CH_2)_2-O]_n$, - C(O)-, where n = 2-6.

A portion of the linker arm may also contain a carbocyclic (or heterocyclic) structure to effect rigidity. One example is a cyclohexyl component as described in Helvetica. Chim. Acta, 1999, 82: 1311-1323.

The linkers on the T and G analogs can be represented as follows:

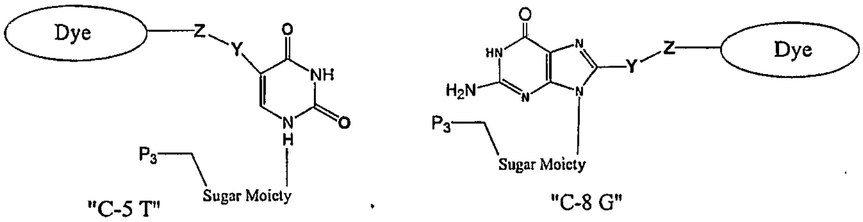

"C-5 T" , "C-8 G"

The linker "Y" on the T and G analogs can be described as follows. The dyes can be attached to the nucleobase via hydrocarbon linkers containing O, S, or N terminal atoms (e.g., J Med. Chem, 1980, 23: 569; Nucleosides, Nucleotides and Nucleic Acids, 1997, 16: 107-114) but are attached preferably through a carbon-carbon covalent bond, preferably containing a terminal alkyne or alkene functionality. (See, for example, U.S. Patent Nos. 5,151,507, 5,608,063, 5,047,519, 5,093,232 and 5,476,928; Russian Chem. Rev. 1999, 68: 483-504; J. Chem. Soc. Chem. Commun. 1994: 1997-8, etc). Additional linkages are described in Nucl. Acids Res. 2001, 29: 1565-1573. The opposite end of the linker Y can have an amine, carboxyl, S or O-containing reactive group appropriate for reactivity with the reactive group Z on the dye thereby forming a stable covalent linkage with group Z. Thus, the two reactive termini of linker Y can be the same or different, as long as they are complementary to the reactive groups on the nucleobase and dye, respectively.

Z is as described above, and can be amino, carboxyl, or an O or S-containing group, as long as Z is a reactive group appropriate for reactivity with the free reactive group on the linker Y after Y is attached to the nucleobase.

Non-limiting examples of the linker Y include:

a) linkers attached through N:

- $NH-(CH_2)_3-O-(CH_2)_2-O-(CH_2)_2-O-(CH_2)_3-NH-$; $-NH-(CH_2)_n-NH-$, where n=2-8; $-NH-(CH_2)_2-O-(CH_2)_2-O-(CH_2)_2-O-(CH_2)_2-O(CH_2)_2-NH-$; and $-NH-[(CH_2)_2-O]_n-NH-$, where n = 2-6.

b) linkers attached through O:

- $O-(CH_2)_3-O-(CH_2)_2-O-(CH_2)_2-O-(CH_2)_3-NH-$; - $O-(CH_2)_n-NH-$, where n=2-8; - $O-(CH_2)_2-O-(CH_2)_2-O-(CH_2)_2-O-(CH_2)_2-O(CH_2)_2-NH-$; and - O $-[(CH_2)_2-O]_n-NH-$, where n = 2-6.

c) linkers attached through S:

- $S-(CH_2)_3-O-(CH_2)_2-O-(CH_2)_2-O-(CH_2)_3-NH-$; - S $-(CH_2)_n-NH-$, where n=2-8; - S $-(CH2)_2-O-(CH_2)_2-O-(CH_2)_2-O-(CH_2)_2-O$ $(CH_2)_2-NH-$; and - S $-[(CH_2)_2-O]_n-NH-$, where n = 2-6.

d) linkers attached through C:

- $(CH_2)_n-NH-$, n=2-15; $-C-C-C(O)-(CH_2)_n-NH-$; n=2-8; $-(CH_2)_n-Q-$, n=2-15 or -C-C-C(O)-Q-, where Q is selected from $-NH-(CH_2)_3-O-(CH_2)_2-O-(CH_2)_2-O-(CH_2)_3-NH-$, $-NH-(CH_2)_n-NH-$(where n=2-12), $-NH-(CH_2)_2-O-(CH_2)_2-O-(CH_2)_2-O-(CH_2)_2-O(CH_2)_2-NH-$, $-NH-[(CH_2)_2-O]$ n -NH- (where n = 2-6), $-(CH_2)_n-NH-$ (where n=2-8), $-(CH_2)_3-O-(CH_2)_2-O-(CH_2)_2-O-(CH_2)_3-NH-$, $-(CH_2)_n-NH-$ (where n=2-8), $-(CH_2)_2-O-(CH_2)_2-O-(CH_2)_2-O-(CH_2)_2-O(CH_2)_2-NH-$, and
- $[(CH2)_2-O]_n-NH-$ (where n = 2-6).

Alternatively, the linker can have a terminal carboxyl (and be attached to an amino group, Z, on the dye). Thus, the linker can be, for example: $-NH-(CH_2)_3-O-(CH_2)_2-O-(CH_2)_2-O-(CH_2)_3-C(O)-$; $-NH-(CH_2)_n-$ C(O)-, where n=2-8; $-NH-(CH_2)_2-O-(CH_2)_2-O-(CH_2)_2-O-(CH_2)_2-O(CH_2)_2-$ C(O)-; and $-NH-[(CH_2)_2-O]_n-$ C(O)-, where n = 2-6. Attachment to

the dye can be through an oximino (Nucleosides, Nucleotides & Nucleic Acids 1999, 18: 979-980).

Preferably Y is selected from: $-C=C-C(O)-(CH_2)_n-NH-$, n=2-8; $-C=C-C(O)-NH-(CH_2)_n-NH-$, n=2-8; and $-C=C-(CH_2)_n-Q-$ (where n=2-8), $-C\equiv C-(CH_2)_n-Q-$ (where n=2-8), or - $C\equiv C-C(O)-Q-$, where Q is selected from $-NH-(CH_2)_3-O-(CH_2)_2-O-(CH_2)_2-O-(CH_2)\ _3-NH-$, - $NH-(CH_2)_n-NH-$ (where n=2-12), $-NH-(CH_2)_2-O-(CH_2)_2-O-(CH_2)_2-O-(CH_2)_2-O\ (CH_2)_2-NH-$, $-NH-[(CH_2)_2-O]_n$ -NH- (where n = 2-6), $-(CH_2)_n-NH-$ (where n=2-8), $-(CH2)_3-O-(CH_2)_2-O-(CH_2)_2-O-(CH_2)_3-NH-$, $-(CH_2)_n-NH-$ (where n=2-8), $-(CH_2)_2-O-(CH_2)_2-O-(CH_2)_2-O-(CH_2)_2-O(CH_2)_2-NH-$, and $-[(CH_2)_2-O]_n-NH-$ (where n = 2-6).

A portion of the linker arm can also contain a carbocyclic (or heterocyclic) structure to effect rigidity. One example is a cyclohexyl component as described in Helvetica. Chim. Acta, 1999, 82: 1311-1323.

Attachment to a fluorescent dye (or other detectable marker) can be through an oximino (Nucleosides, Nucleotides & Nucleic Acids 1999, 18: 979-980). Alternatively, the linker can have a terminal carboxyl (and be attached to an amino group, Z, on the dye). The following are non-limiting examples of this arrangement: $-NH-(CH_2)_3-O-(CH_2)_2-O-(CH_2)_2-O-(CH_2)_3-C(O)-$, $-NH-(CH_2)_n\ C(O)-$ n=2-8, also $-NH-(CH_2)_2-O-(CH_2)_2-O-(CH_2)_2-O-(CH_2)_2-O(CH_2)_2\ C(O)-$, $-NH-[(CH_2)_2-O]_n-\ C(O)-$ n = 2-6; other linkers described herein above or known in the art can also comprise a terminal carboxyl for the same purpose.

The effects of linkers attached to deoxyuridine (dU) residues on oligonucleotide hybidization is described in Bull. Chem. Soc. Jpn 1995, 68: 1981-1987. The effects described provide guidance to one skilled in the art regarding the design and placement of linkers onto dU residues such that they continue to permit oligonucleotide hybridization.

Linker L-2 can be described as -(Z)-(Y)- (as shown below; wherein L-2 is -(Z-Y)-) wherein Z represents a reactive functional group on the quencher capable of reacting with Y to

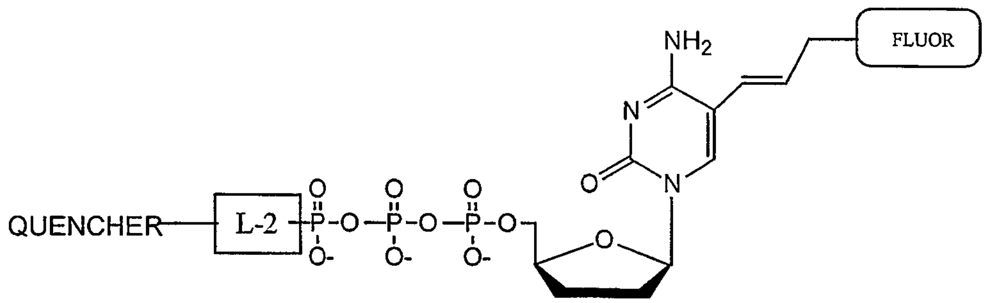

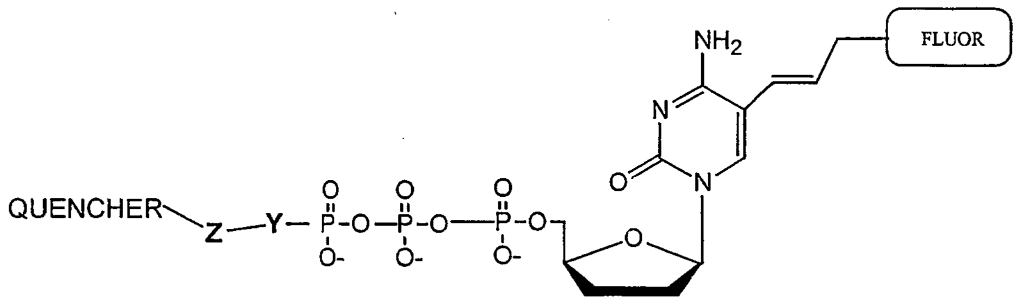

form a stable linkage. Y, in turn, forms a covalent bond with the γ-phosphorus atom.

The Y component of L-2 thus demonstrates dual functionality. That is, facilitating attachment to Z at one terminus and to the γ-phosphorus atom at the other terminus.

In one embodiment, linkers (L-2) can be made from α-hydroxy-ω-amines ($H_2N-(CH_2)_n\ OH$). In this case Y = $-NH-(CH_2)_n-O-$ and Z = a carbonyl group. The NH group of Y forms an amide linkage with the carboxyl group (Z) on a quenching moiety. The oxygen terminus of Y forms a covalent bond with the γ-phosphorus atom effecting a phosphate ester linkage.

The structure represented by Y can be made from a variety of α-hydroxy-ω-amines including: ethanolamine, or any compound of the formula: $H_2N-(CH_2)_n-OH$, n = 1-12. Thus, Y can be chosen from (but not limited to) the following:

$HN\ -(CH_2)_3-O-(CH_2)_2-O-(CH_2)_2-O-(CH_2)_3-NH-$, $HN\ -(CH_2)_n-O-$n=2-8, also

$HN\ -(CH_2)_2-O-(CH_2)_2-O-(CH_2)_2-O-(CH_2)_2-O(CH_2)_2-O-$, $-NH-[(CH_2)_2-O]_n$ -On = 2-6.

Alternatively, Y can have a terminal carboxyl (and be attached to an amino group, Z, on the quencher). Thus:

- HN $-(CH_2)_3$-O-$(CH_2)_2$-O-$(CH_2)_2$-O-$(CH_2)_3$-C(O)-, - HN $-(CH_2)_n$-C(O)-n=2-8, also - HN $-(CH_2)_2$-O-$(CH_2)_2$-O-$(CH_2)_2$-O-$(CH_2)_2$-O $(CH_2)_2$- C(O)-, -NH-[$(CH_2)_2$-O]$_n$- C(O)- n = 2-6.

A portion of the linker arm may also contain a carbocyclic (or heterocyclic) structure to effect rigidity. One example is a cyclohexyl component as described in Helvetica. Chim. Acta, 1999, 82, 1311-1323.

Additional alternatives to Y include structures that contain nitrogen, sulfur or carbon atoms that bond with the terminal γ-phosphorus atom (N, S or $CH_n$ in place of the terminal oxygen atom). Thus, Y can be chosen from (but not limited to) the following:

(Linkers that are attached to the γ-phosphorus atom via a terminal amine, N-attached):

- HN $-(CH_2)_3$-O-$(CH_2)_2$-O-$(CH_2)_2$-O-$(CH_2)_3$-**NH**-, -HN-$(CH_2)_n$- **NH** - n=2-8, also
- HN $-(CH_2)_2$-O-$(CH_2)_2$-O-$(CH_2)_2$-O-$(CH_2)_2$-O$(CH_2)_2$-**NH** -, -HN-[$(CH_2)_2$-O]$_n$**NH-**n = 2-12.

(Linkers that are attached to the γ-phosphorus atom via a terminal thiol, S-attached):

- HN $-(CH_2)_3$-O-$(CH_2)_2$-O-$(CH_2)_2$-O-$(CH_2)_3$-**SH**-, - HN $-(CH_2)_n$-**SH** - n=2-8, also
- HN $-(CH_2)_2$-O-$(CH_2)_2$-O-$(CH_2)_2$-O-$(CH_2)_2$-O$(CH_2)_2$- **SH** -, -HN-[$(CH_2)_2$-O]$_n$**SH-**n = 2-12.

(Linkers that are attached to the γ-phosphorus atom via a terminal carbon atom,[phophonate linkage], Carbon-attached):

- HN $-(CH_2)_3$-O-$(CH_2)_2$-O-$(CH_2)_2$-O-$(CH_2)_3$-(**CH**)$_n$-, - HN -(CH2)$_n$- (**CH**)$_n$- n=2-8, also - HN $-(CH_2)_2$-O-$(CH_2)_2$-O-$(CH_2)_2$-O-$(CH_2)_2$-O$(CH_2)_2$-(**CH**)$_n$-, -HN-[$(CH_2)_2$-O]$_n$(**CH**)$_n$-n = 2-12.

*Fluorescent Moiety*

Any fluorescent molecule that can be attached to a nucleotide can be used as a fluorescent moiety according to the invention. Fluorescent dyes useful according to the invention will permit the recognition and incorporation of the attached nucleotide by a nucleic acid polymerase. Standard nucleotide incorporation assays known in the art can be used to test whether a given fluorescent moiety is compatible with recognition and incorporation by a nucleic acid polymerase enzyme. "Fluorescent moieties" which are useful in the present invention include, but are not limited to 4-acetamido-4'-isothiocyanatostilbene-2,2'-disulfonic acid, acridine, acridine isothiocyanate, 5-(2'-aminoethyl)aminonaphthalene-1-sulfonic acid (EDANS), 4-amino-N-[3-vinylsulfonyl)phenyl]naphth- alimide-3,5 disulfonate, N-(4-anilino-1-naphthyl)maleimide; anthranilamide, BODIPY, Brilliant Yellow, coumarin, 7-amino-4-methylcoumarin (AMC, Coumarin 120),7-amino-4-trifluor- omethylcouluarin (Coumaran 151), cyanine dyes, cyanosine, 4',6-diaminidino-2-phenylindole (DAPI), 5',5"-dibromopyrogallol-sulfonaph- thalein (Bromopyrogallol Red), 7-diethylamino-3-(4'-isothiocyanatophenyl)-- 4-methylcoumarin, diethylenetriamine pentaacetate, 4,4'-diisothiocyanatodi- hydro-stilbene-2,2'-disulfonic acid, 4,4'-diisothiocyanatostilbene-2,2'-di- sulfonic acid, 5-[dimethylamino]naphthalene-1-sulfonyl chloride (DNS, dansylchloride), 4-dimethylaminophenylazophenyl-4'-isothiocyanate (DABITC), eosin, eosin isothiocyanate, erythrosin, erythrosin B, isothiocyanate, ethidium, fluorescein, 5-carboxyfluorescein (FAM),5-(4,6-dichlorotr- iazin-2-yl)aminofluorescein (DTAF), 2',7'-dimethoxy-4'5'-dichloro-6-carboxyfluorescein (JOE), fluorescein, fluorescein isothiocyanate, QFITC, (XRITC), fluorescamine, IR144, IR1446, Malachite Green isothiocyanate, 4-methylumbelliferoneortho cresolphthalein, nitrotyrosine, pararosaniline, Phenol Red, B-phycoerythrin, o-phthaldialdehyde, pyrene and derivatives: pyrene, pyrene butyrate, succinimidyl 1-pyrene, butyrate quantum dots, Reactive Red 4 (Cibacron.TM. Brilliant Red 3B-A) rhodamine, 6-carboxy-X-rhodamine (ROX), 6-carboxyrhodamine (R6G), lissamine rhodamine B sulfonyl chloride rhodamine (Rhod), rhodamine B, rhodamine 123, rhodamine X isothiocyanate, sulforhodamine B, sulforhodamine 101, sulfonyl chloride derivative of sulforhodamine 101 (Texas Red), N,N,N',N'-tetramethyl-6-carboxyrhodamine (TAMRA), tetramethyl rhodamine, tetramethyl rhodamine isothiocyanate (TRITC), riboflavin, rosolic acid, Cy 3, Cy 5, Cy 5.5, Cy 7, IRD 700, IRD 800, La Jolla Blue, phthalo cyanine, Oregon green, and naphthalo cyanine.

In one aspect, the fluorescent dye useful according to the invention encompasses the Big Dye™ technology of Perkin-Elmer Applied Biosystems. The Big Dye™ dyes comprise a fluorescein donor dye (e.g., 6-carboxyfluorescein) linked directly to one of four different dichlororhodamine acceptor dyes. The dichlororhodamine acceptor dyes are each excited by light in the emission spectrum of the fluorescein donor dye, but emit at distinguishable wavelengths. In this arrangement, excitation at a single wavelength (the excitation maximum for the fluorescein dye) results in emission at one of four different wavelengths, depending upon the identity of the acceptor dye. In this aspect of the invention, then, there are three moieties involved in determining the fluorescence behavior of the nucleotide. The two-dye (e.g., Big

Dye™) fluorescent moiety configuration is paired with a quencher to generate a dual labeled nucleotide according to the invention. That is, the two-dye structure is used as the "fluorescent moiety," and a quencher of the fluorescence emitted by that two-dye structure is used as the "quencher moiety." This configuration is diagrammed below:

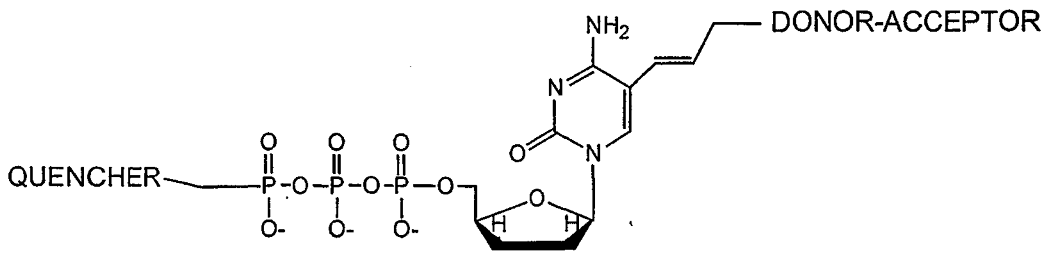

In one aspect, a fluorescent moiety useful in the present invention is a fluorescent dye that emits fluorescent energy at a wavelength that can be distinguished by fluorescent detection equipment (for example, the ABI Prism™ 377 Sequencer) when two or more such dyes are present in one sample, e.g., in a sequencing reaction. An example of a set of spectrally distinguishable fluorescent dyes useful for nucleotide sequencing reactions is rhodamine-6G (R6G), rhodamine 110 (R110), tetramethyl rhodamine (TAMRA) and rhodamine X (ROX) (available from Molecular Probes, Eugene, OR). Another set of other spectrally distinguishable fluorescent dyes include, for example, the variants dichloro-R6G, dichloro-ROX, dichloro-R110 and dichloro-TAMRA (Applied Biosystems, Inc., Foster City, CA).

In one aspect of the invention, the fluorescent moiety or fluorophore is attached to the nucleobase. However, it can be useful to attach the fluorophore to other portions of the nucleotide molecule, e.g., to the "sugar" moiety, or to the α-phosphate (attachment to the α-phosphate can be achieved chemically, but it is not clear that such a nucleotide would represent a functional substrate for enzymatic incorporation into a polynucleotide). What is critical is that the fluorescent moiety is attached close enough to the quencher moiety to achieve at least a 2-fold quenching efficiency. As discussed herein above, this distance will vary with the identity of the fluorescent moiety: quencher pair, but is based upon the Förster radius, calculated as described herein and known in the art. The distance will generally be in the range of 10 to 100 A. One skilled in the art can readily determine whether the selected sites of attachment of the quencher and fluorescent moiety, respectively, will provide the desired (2-fold or greater, preferably greater) degree of quenching. The typical Förster radius for a non-limiting selection of donor:quencher pairs is given in Table 1.

Figure 3 shows examples of dual labeled nucleotides according to the invention wherein the fluorescent moiety is attached to the "sugar" moiety. Dyes can be attached to a 3' amino group of nucleotides following the method of R.S. Sarfati (Proc. Nat. Acad. Sci USA 1995, 92, 10859-10863) or Engels, (Bioorg. Med. Chem Lett. 1994, 4, 1975-1978). Nucleotides with 3' thiol or amino groups can be made according to US Pat 5,679,785 and labeled with amino-reactive or thiol-reactive dyes following established procedures. Figure 4 shows examples of dual labeled uncleotides according tot he invention wherein the fluorescent moiety is attached to the α-phosphate moiety.

<u>Synthesis of Dual-labeled Nucleotide Analogs</u>

Standard nucleosides and nucleotides are available from a variety of sources. Variant nucleosides useful for dual-labeled nucleotides of the invention are widely described in the literature and can be synthesized according to methods known in the art. As examples, synthetic approaches to the synthesis of representative acyclic phosphonomethoxyethyl (PME) nucleosides, fluorescently labeled on their nucleobase moieties, are described below. Methods of attaching a quencher moiety to the polyphosphate of such nucleosides are also described below.

A. Synthetic approaches to the synthesis of representative acyclic nucleosides.

The synthesis of PME-A is described by Holy & Rosenberg, 1987, Collect. Czech. Chem. Commun. 52: 2775, Holy & Rosenberg, 1987, Collect. Czech. Chem. Commun. 52: 2801, Holy et al., 1990, supra, and Starrett et al., 1994, J. Med. Chem. 37: 1857-1864, each of which is incorporated herein by reference. The general synthesis of nucleoside 5'-triphosphates is described by Moffatt, 1964, Can. J. Chem. 42: 599, which is also incorporated herein by reference.

The PME nucleoside analogs can be made using precursors and synthetic pathways known in the art. For example, the precursor 2-chloroethoxymethyl chloride was made following J. Heterocycl. Chem., 2000, 37: 1187-91 and converted to diethy-2-chloroethoxymethylphosphonate as described (Collect. Czech. Chem. Commun. 1989, 54: 2190), or converted to di-(2-propyl)-2-chloroethoxymethylphosphonate as described in J. Heterocycl. Chem., 2000, 37: 1187-91. Alkylations (attachment of the nuceobases) are performed according to the general procedures described in J. Med. Chem. 1999, 42: 2064-2086.

The synthesis of a PME-A nucleotide analog fluorescently labeled on the adenine nucleobase can be performed as follows. Di-(2-propyl)-2-chloroethoxymethylphosphonate is reacted with 6-chloropurine in the presence of sodium hydride to effect attachment at the N-9 position as described (J. Med. Chem. 1999, 42: 2064-2086). Either of two routes can then be followed. Preferably, (Route A) diester hydrolysis is effected using bromotrimethylsilane. Pyrophosphorylation according to the procedure of Moffat (Can.J. Chem. 1964, 42, 599-specifically described in Collect. Czech. Chem. Commun. 1987, 52, 2801-9), to yield the diphosphate derivative (triphosphate analog). Displacement of the 6-chloro group with 4,7,10-trioxatriundecane-1,13-diamine provides the requisite linker attachment to the purine base. Coupling of the dye is then accomplished in aqueous sodium borate buffer using an activated ester (N-hydroxysuccinimide) derivative of the fluorescent dye to give the desired fluorescently labeled PME-A-pp Analog.

Route B, which can be followed if the 6-chlor group proves too labile in the pyrophosphorylation procedure, is as follows. After monoester hydrolysis of the phosphonate group, the 6-chloro group is displaced with 4,7,10- trioxatriundecane-1,13-diamine and the amine terminus is blocked by reaction with BOC anyhdride. The remaining phosphate ester is then deprotected using bromotrimethylsilane. Pyrophosphorylation can then be performed as described in Route A, followed by deprotection of the Fmoc functionality using aqueous morpholine. Coupling of the dye as described in Route A will then yield the desired PME-A-pp analog fluorescently labeled on the adenine nucleobase.

The synthesis of a PME-C nucleotide analog fluorescently labeled on the nucleobase can be performed as follows. 4-Methoxy pyrimidin-2-one is synthesized as described (Nucl. Acids. Res. 1973: 19-34), or by a more straightforward route (e.g., that taught in US patent 5,359,067) from uracil via its 4-(1,2,4-triazolide) derivative. Reaction with di-(2-propyl)-2-chloroethoxymethylphosphonate effects alkylation an the N-1 position of the pyrimidine ring as described (Collect. Czech. Chem. Commun. 1989, 54: 2190-2209). Following monoester hydrolysis of the phosphonate group using lithium azide, the 4-methoxy group is displaced with 4,7,10- trioxatriundecane-1,13-diamine and the amine terminus is subsequently blocked by reaction with BOC anhydride or pixyl chloride. The remaining phosphate ester is then deprotected using bromotrimethylsilane. Pyrophosphorylation, deprotection and dye coupling are then performed as described above (in the synthesis of the PME-A analog) to yield the PME-C-pp analog fluorescently labeled on the nucleobase.

The synthesis of a PME-T nucleotide analog fluorescently-labeled on the nucleobase can be performed as follows. 5-Iodo-4-methoxy pyrimidin-2-one is synthesized starting with 5-iodouracil. Alkylation with di-(2-propyl)-2-chloroethoxymethylphosphonate is then accomplished as described for the PME-C analog, above. Reaction of N-1-phosphonmethoxyethyl-5-iodo-4-methoxy pyrimidin-2-one- with Fmoc-protected propargylamine in the presence of a palladium(0) catalyst (J. Org. Chem. 1989: 54, 3420-3422) provides for attachment of a propargylamine linker at the pyrimidine C-5 position. Reaction with bromotrimethylsilane effects ether cleavage of the 4-methoxy group and concommitant phosphonate diester hydrolysis to give the Fmoc-protected propargylamine derivative of PME-T. Pyrophosphorylation, deprotection and dye coupling are then accomplished as described above (in the synthesis of the PME-A analog) to yield the PME-T-pp analog fluorescently labeled on the nucleobase.

The synthesis of a PME-Gpp nucleotide analog (7-deazaguanine analog) is performed as follows. Di-(2-propyl)-2-chloroethoxymethylphosphonate is be reacted with 6-chloro-7-deazaguanine in the presence of cesium carbonate as described for the cytidine analog. Regiospecific iodination at the 7-position is accomplished as reported by Balow, G in Nucleosides, Nucleotides (1977) 16, 941-944. Propargylamine linker attachment, deprotection, phosphorylation and labeleing are accomplished as described for the fluorescent PME-T analog (described above) to yield the fluorescently labeled PME-G-pp analog.

Quencher, e.g., Dabcyl, is added to PME-Gpp analog or to dye-labeled 7-deaza-dGTP (e.g., Rox-labeled 7-deaza-dGTP or Rox-labeled 7-deaza-ddGTP (NEN, Amersham)) as described in section B, "Attachment of quencher moiety to the polyphosphate group," below. The $\gamma$-phosphate of the Rox-labeled 7-deaza-dGTP (or -ddGTP) is activated according to established methods, e.g., those of Kao et al., 2000, Virology 274: 429-437 or.Rychlik et al., 1982, Biochim. Biophys. Acta 698: 116-127), followed by reaction with Dabcyl amine (or Dabsyl amine) as described below.

B. Attachment of quencher moiety to the polyphosphate group.

A standard reaction for the attachment of a quencher moiety to the gamma phosphate of a nucleoside useful according to the invention will proceed as follows. The monosodium salt of the fluorescent nucleoside triphosphate (0.3 mmol) is dissolved in 2 ml of 0.1 M triethylamine bicarbonate (soln. A) and passed over a column of Ag 50W-X8 cation exchange resin equilibrated in soln. A. The column is washed with 25-50 ml of soln. A and the eluate concentrated to dryness. Five ml of anhydrous methanol is added to the residue, evaporated to dryness and this procedure repeated twice more and the residue dried under high vacuum for 30 min. The residue is dissolved in 5 ml of DMSO, then 3 mmol of dicyclohexyl carbodiimide(DCC) is added and the mixture stirred under nitrogen overnight. One to 3 mmol of dabsyl amine (described below) is added and stirred under nitrogen overnight. The solution is diluted with 20 ml of water and acidified to pH 5 with acetic acid, reduced in volume to 5 ml by rotary evaporation and purified by preparative HPLC and acetonitrile gradient elution.

Dabsyl amine is synthesized by mixing 6 mmol of 4-dimethylaminoazobenzene-4'-sulfonyl chloride with a 3 to 5 mmol excess of 1,6-diaminohexane in 100 ml of dry dimethyl formamide for 2 hours at room temperature. The reaction contents are extracted with ethyl acetate, the solvent is evaporated and the product is kept dry during storage.

In addition to the above-described methods of attaching a quencher to the γ phosphate moiety, attachment of a dye to the γ-phosphate through a *sulfur* atom (γ-thiophosphate) using thiol-reactive dyes is described by Brenner, 2000, J. Biol Chem 275: 4555-4560. See also: Eckstein, editor, Oligonucleotides and Analogues: A Practical Approach (IRL Press, Oxford, 1991); Zuckerman et al., 1987, Nucleic Acids Research, 15:5305-5321 (3'thiol group on oligonucleotide); Sharma et al., 1991, Nucleic Acids Research 19: 3019 (3' sulfhydryl); Giusti et al., 1993, PCR Methods and Applications 2:223-227 and Fung et al., U.S. Pat. No. 4,757,141. Synthesis of NTPs containing reactive amino groups at the γ-phosphate is described by Blakley, 1975, BioChemistry 14: 4804-4812, and Nelson et al., 1989, Nucleic Acids Research 17:7187-7194 (3' amino group). Attachment of the quencher (in the form of its succidimidyl ester) to the amine group is effected following established procedures. Synthesis of NTPs with various groups attached to the γ-phosphate through an *oxygen* atom (γ-phosphate ester) is described by Krayavsky et al., 1996, J. Biol Chem 271: 24389-24394. Felicia et al. describe the synthesis and spectral properties of a (γ-1,5-EDANS)ATP(see, Felicia et al., 1986, Arch. Biochem Biophys., 246: 564-571 (1986)), and Sato et al. disclose a homogeneous enzyme assay that uses a fluorophore moiety (bimane) attached to the γ-phosphate group of the nucleotide and a quencher moiety attached to the 5-position of uracil.

Nucleotides can be derivatized to express terminal thiol groups at the γ-phosphate using cystamine dihydrochloride as described below for dUTP. A dual labeled nucleotide can be made starting with Fluorescein-12-dUTP (available from Stratagene) by substituting Fluorescein-12-dU for dUTP in the procedure described below. Nucleotides derivatized with cystamine are suitable for attachment to quenchers or dyes through the thiol group following established methods (e.g., using BODIPY TR-iodoacetamide (commercially available from Molecular Probes D-6011; protocol for attachment supplied with the reagent and summarized below).

Procedure for the Synthesis of dUTP-thiol

dUTP was dissolved in 1 mL of 0.1 M MES pH 5.7 (Sigma M 3023) and adjusted to pH 5.75. Cystamine dihydrochloride (10 mg, 44 .mu.mol; Sigma C 8707) was dissolved in 2.5 mL of 0.1 M MES pH 5.7 and adjusted to pH 5.75. EDC (9 mg, 47 .mu.mol; Pierce 22980) was dissolved in 0.5 mL of 0.1 M MES pH 5.7 and was added immediately to the dUTP solution. After 10 minutes, the cystamine solution was added and the pH was maintained between 5.5 and 5.8 while the reaction proceeded at room temperature. After two hours, the pH was adjusted to 7.0 and the sample was stored at -20.degree. C. The product was purified by reversed-phase HPLC.

Attachment of BODIPY-TR to dUTP-thiol

dUTP-thiol (from above) was dissolved in 5.4 mL of 5 mM TCEP (Pierce 20490), 30 mM sodium phosphate adjusted to pH 7.5. BODIPY TR-iodoacetamide (5mg, 7.4 umol; Molecular Probes D-6011) was dissolved in 2.6 mL of N,N-dimethylformamide and was added to the dUTP-thiol solution. After standing at room temperature in the dark for 5 hours, the product was purified by reversed-phase HPLC.

Detection of Dual-labeled Nucleotide Analogs

The present invention relates to dual-labeled nucleotide analogs which, prior to incorporation into a poly-or oligonucleotide, do not emit a fluorescent signal because the quencher moiety is closely apposed to the fluorescent moiety. Upon incorporation of the dual-labeled nucleotide analog into a growing poly- or oligonucleotide chain by a nucleic acid polymerase enzyme, a pyrophosphate moiety is cleaved off of the analog along with the quencher moiety, which is attached to the most distal (the γ-phosphate in a triphosphate moiety) phosphate from the sugar moiety. Removal of the quencher moiety in this manner permits the fluorescent moiety to emit light energy when contacted with light of the appropriate excitation wavelength.

Detectors used with the dual-labeled nucleotide analogs according to the invention can vary, and include any suitable detectors for detecting optical changes in labeled nucleotides. In one embodiment of the invention, a oligonucleotide with an incorporated dual-labeled nucleotide analog is excited by a light source in communication with the biosensor. In a further embodiment, recognition of a target molecule (i.e., oligonucleotide with an incorporated analog) will cause a large increase in fluorescence emission over a low background signal. The high signal to noise ratio permits small signals to be measured using high-gain detectors.

Light sources include, but are not limited to, filtered, wide-spectrum light sources, laser light sources, such as Argon-Ion lasers (emitting at nine discrete spectral lines between 458 and 530) a Helium Neon laser (emitting at 633 nm), and a diode laser (emitting at 635 nm). Excitation wavelengths and emission detection wavelengths will vary

depending on the fluorescent moieties used, and are well known to those of skill in the art.

In one embodiment of the disclosure, detection of changes in the optical properties of the fluorescent moiety linked to the nucleotide analog is performed using any of a cooled CCD camera, a single-photon-counting detector, or other light sensitive sensor. In one embodiment of the invention, the detector is optically coupled to the target activated biosensor through a lens system, such as in an optical microscope (e.g., a confocal microscope). In another embodiment of the invention, a fiber optic coupler is used, where input to the optical fiber is placed in close proximity to the nucleotide analog.

In still another embodiment of the disclosure, the fluorescence emitted by an incorporated dual-labeled nucleotide analog is determined using a spectrometer (e.g., such as a luminescence spectrometer) and fluorescence intensity is measured at emission wavelengths appropriate for the fluorescent moiety used (e.g., acridine at 495 nm; fluorescein at 515 nm). In one embodiment of the invention, an oligonucleotide molecule with an incorporated dual-labeled nucleotide analog is in solution and is pipetted into a cuvette within the spectrophotometer.

In embodiments of the invention where the dual-labeled nucleotide analogs of the invention are attached to substrates (i.e., incorporated into an oligo- or polynucleotide which is stably associated with a solid substrate), such as a glass slide or microarray, it is desirable to reject any stray or background light to permit the detection of very low fluorescence signals. In one embodiment of the invention, a small sample volume is probed to obtain spatial discrimination by using appropriate optics, such as evanescent excitation or confocal imaging. Furthermore, background light can be minimized by the use of wavelength filters between the sample and the detector and by using light shielding to remove any ambient light from the measurement system.

In this embodiment, spatial discrimination in a direction normal to the interface of the substrate is obtained by evanescent wave excitation. Evanescent wave excitation uses energy that propagates into the lower-index of refraction medium when an electromagnetic wave at the interface is totally internally reflected into a higher-refractive index material. A laser beam is propagated at an angle larger than the critical angle for total internal reflection, into a prism or a optical fiber. Target recognition is detected by a change in the fluorescent emission of the nucleic acid sensor. This confines the excitation to within several hundred nanometers of the surface. Spatial discrimination in the plane of the interface is achieved by the optical system described above.

In another embodiment of the disclosure, the detection system is a single photon counter system (see, e.g., U.S. Patent Number 6,016,195 and U.S. Patent Number 5,866,348).

<u>Nucleic Acid Sequencing:</u>

Nucleic acid sequencing methods are widely known in the art. Methods of particular importance to the invention are those based upon the enzymatic incorporation of a chain terminator into the growing polynucleotide chain. The original chain terminator method was described by Sanger, et al. (1977, Proc. Natl. Acad. Sci. U.S.A., 74: 5463-5467). In this method, a single-stranded template nucleic acid is sequenced by using a nucleic acid polymerase to synthesize a set of polynucleotide fragments wherein the fragments (a) have a sequence complementary to the template nucleic acid sequence, (b) differ in length by a single nucleotide, and (c) have a 5'-end terminating in a known nucleotide, e.g., A, C, G, or T. In the Sanger method, an oligonucleotide primer is hybridized to the template nucleic acid, and the 3'-end of the primer serves as an initiation site for polymerase-mediated polymerization of a complementary polynucleotide fragment. The primer extension reaction comprises contacting the annealed template-primer hybrid with the four deoxynucleotides (dA, dC, dG and dT), a nucleic acid polymerase enzyme, and one nucleotide chain terminator (Sanger's method called for 2',3'-dideoxynucleotide triphosphate chain terminators). The incorporation of the dideoxy terminator forms a primer extension product which lacks a hydroxy group at the 3'-terminus and thus can not be further extended by the polymerase. Four separate primer extension reactions are performed, each including a single terminator corresponding to one of dA, dC, dG and dT. The competition between the dNTP and its corresponding terminator for incorporation by the polymerase results in a distribution of different-sized extension products, each extension product terminating with the particular terminator used in the reaction. Electrophoretic separation of the four separate reactions in parallel produces a "ladder" of extension fragments, each starting with the annealed primer common to all reactions and ending with one of the four terminators used. The sequence of the complement (and thereby the sequence of the template) is read directly from the order of fragments on the gel.

Numerous variations on the Sanger method are known to those skilled in the art. The fragments generated in the sequencing reactions were originally detected through use of radiolabel ($^{32}P$ or $^{35}S$) incorporated either into the primer or into one of the dNTPs. More recently, detection has been achieved by labeling each terminator with a fluorescent dye (see e.g., Prober et al., Science, 238: 336-341 (1987); and U.S. Pat. No. 5,151,507). The use of fluorescent dyes overcomes problems related to the limited shelf life of radiolabeled products and the difficulties in handling, storing and disposing of radioactive wastes generated in the process. As noted above, the use of four spectrally distinguishable fluorescent dyes, one on each terminator, also permits the sequencing reaction to be performed in a single tube or vessel, instead of the four tubes necessary for the original method.

Other variations on the Sanger process include the use of thermostable nucleic acid polymerase enzymes, and "cycle sequencing", which is essentially a PCR reaction performed in the presence of a chain terminator. Among other advantages, thermostable polymerases and cycle sequencing increase the sensitivity of the reactions by reducing the amount of starting template needed and overcome the need for single-stranded template molecules.

Dual-labeled nucleotide analogs which are also chain terminators according to the invention can be used in a sequencing or minisequencing protocol in place of dideoxynucleotide chain terminators commonly used in the art. A chain terminating dual-labeled nucleotide analog has a sugar moiety which is, or is equivalent to a 2',3'-dideoxypyrofuranose molecule. The dual-labeled nucleotide analogs of the present invention have the advantage of reduced background fluorescence compared with more traditionally labeled chain terminating nucleotide analogs. Since the dual-labeled nucleotide analogs of the present invention do not emit a fluorescent signal unless they are incorporated into a polynucleotide chain, background fluorescence resulting from unincorporated analogs is significantly reduced.

Nucleic acid sequencing methods using the dual-labeled nucleotide analogs according to the invention will generally have the scheme where an oligonucleotide primer is annealed to a sequencing template polynucleotide, and the annealed primer/template complex is contacted with a nucleic acid polymerase enzyme in the presence of a dual-labeled nucleotide analog, under conditions and for a time sufficient to permit extension of the primer by the polymerase. Incorporation of the dual-labeled nucleotide analog, and corresponding de-quenching of the fluorescent moiety under these conditions permits the determination of nucleic acid sequence information about the template polynucleotide because the analog is only incorporated where it is the complement of a template nucleotide. Incorporation is generally detected following size separation of the extension products, but can also be measured without such size separation, as in the minisequencing methods (see below). Reactions will most often include deoxynucleotide triphosphates in addition to one or more dual-labeled nucleotide analog chain terminators, but variations such as the minisequencing methods do not necessarily require this.

As noted above, the so called "minisequencing" techniques also benefit from the dual-labeled nucleotide analogs according to the invention. Minisequencing generates limited sequence information, most often information about a single nucleotide. Minisequencing techniques have become increasingly important as researchers and clinicians seek to determine the genotypes of individuals with respect to polymorphisms and mutations (see, e.g., Syvanen et al., 1990, Genomics, 8:684-692; Makridakis & Reichardt, 2001, Biotechniques 6:1374-1380). There are numerous variations on the technique, but the basic premise is that a primer is annealed so that its 3' end is hybridized to the template nucleotide immediately adjacent to the nucleotide one wishes to identify. The annealed template is then exposed to a nucleic acid polymerase (e.g., Taq polymerase) and a labeled chain terminator nucleotide triphosphate analog, followed by detection of incorporated label. If the analog is incorporated by the polymerase enzyme, the unknown nucleotide is identified as the complement of the nucleobase of the analog. If the analog is not incorporated, the process is repeated with chain terminator analogs for each of the three remaining nucleotide triphosphates until one is incorporated, thereby identifying the template nucleotide. In one important variation, four different fluorescently labeled chain terminator analogs are included in the same reaction, one corresponding to each of dA, dC, dG and dT, followed by detection of incorporated fluorescence. If the four analogs are labeled with spectrally distinguishable fluorophores, the identity of the target nucleotide can be identified from a single reaction.

The minisequencing techniques are particularly well adapted for microarray-based or other solid phase (e.g., microbead) analysis. When the methods are performed on a microarray, target fragments (most often PCR generated fragments) are immobilized on the array, followed by the application of the minisequencing protocol and detection on the microarray. Examples of these approaches are described by Huber et al., 2001, Anal. Biochem. 299: 24-30 and Shapero et al., 2001, Genome Res. 11: 1926-1934.

<u>Functional Testing of Dual-labeled Nucleotides Useful According to the Invention</u>

Dual-labeled nucleotide analogs useful according to the invention can be tested for their ability to be recognized and incorporated by a nucleic acid polymerase, and for their ability to act as chain terminators, as follows.

Generally, chain terminator function is tested by setting up a standard primer extension assay and running the assay in the presence and absence of the nucleotide analog. A standard assay will involve, for example, a template nucleic acid molecule in which at least one nucleotide is the complement of the nucleobase carried by the dual-labeled nucleotide analog, an oligonucleotide primer that hybridizes to the template , and a nucleic acid polymerase. The primer is annealed to the template in a buffer compatible with the function of the nucleic acid polymerase, followed by the addition of the polymerase and the labeled dual-labeled nucleotide analog (plus any conventional nucleotides necessary for primer extension up to a template nucleotide complementary to the dual-labeled nucleotide being tested). Reactions are incubated at a temperature compatible with activity of the enzyme, and reaction products are separated on a polyacrylamide gel, followed by detection of incorporated label. Alternatively, following the primer extension reaction, incorporation of labeled dual-labeled nucleotide analog can be measured by trichloroacetic acid (TCA) precipitation of the reaction products. Buffers and reaction temperatures are well known in the art for a wide variety of nucleic acid polymerase

molecules. If label is incorporated, the dual-labeled nucleotide analog serves as a substrate for the polymerase. The fidelity or specificity of incorporation by the polymerase can be further analyzed through the use of specialized templates that, for example, do not have nucleotides complementary to the nucleobase on the analog.

Useful template nucleic acid molecules can exist in a variety of forms, e.g., a single stranded DNA, such as that isolated from an M13 bacteriophage, a plasmid, or a DNA fragment generated by PCR or restriction digest.). Homopolymers, simple repeats (e.g., AGAGAGAGA...) and templates either devoid of or rich in a given nucleotide are also useful, especially to evaluate the fidelity or specificity of incorporation. Example 1 below describes one set of conditions for evaluation of incorporation and chain termination effects of a dual-labeled nucleotide analog.

Chain termination by a dual-labeled nucleotide analog according to the invention can be measured by conducting two primer extension reactions containing all four dNTPS (dG, dA, dT, and dC), one reaction with and one reaction without the nucleotide analog. Following the primer extension reaction, reaction products are separated electrophoretically and visualized on the basis of an incorporated label (e.g., attached to the primer, or included as a labeled dNTP). Chain termination is evident if the reaction products made in the presence of the chain terminator are shorter on average than those in its absence, or form a "ladder," where each discretely sized fragment making up the ladder represents a primer extension event terminated after incorporation of the nucleotide analog.

The efficiency of recognition of a given dual-labeled nucleotide analog by a given polymerase will influence its usefulness as a chain terminator in nucleic acid sequencing reactions. The effectiveness of a given chain terminator in a reaction catalyzed by a given polymerase enzyme depends upon the $K_d$ of the enzyme-analog interaction. That is, the equilibrium binding constant of the enzyme and the analog determines how much of a given terminator is necessary to bring about chain termination in a reaction also containing non-terminator nucleotides (e.g., deoxynucleoside triphosphates). Generally, the less efficient the interaction, the higher the ratio of chain terminator to non-terminating nucleotide necessary to bring about efficient chain termination.

In order to evaluate the efficiency of recognition of a particular dual-labeled nucleotide analog, one can vary the ratio of deoxynucleotide to nucleotide analog in the primer extension reaction. For example, the ratio of nucleotide analog to deoxynucleotide can be varied over a range from 1:50, to 1:10, to 1:1, to 1:1, to 5:1 and 10:1. If these titration reactions are performed alongside reactions with similar ratios of the corresponding conventional dideoxyNTP, the efficiency of recognition/termination for the polymerase is determined relative to the dideoxyNTP. While it is preferred that a dual-labeled nucleotide analog useful according to the invention will be effective at or below the concentration of the corresponding ddNTP, one skilled in the art can readily adjust the ratios of different chain terminators in order to achieve sequencing results similar to or superior to those achievable using conventional ddNTPs.

Table 1- Typical Förster radius ($R_0$) values for selected dye/quencher pairs

| Fluorescence donor | Acceptor/Quencher | | | | | | | | | |
|---|---|---|---|---|---|---|---|---|---|---|
| | Alexa Fluor 488 | Alexa Fluor 546 | Alexa Fluor 555 | Alexa Fluor 568 | Alexa Fluor 594 | Alexa Fluor 647 | QSY 35 | Dabcyl | QSY 7 QSY 9 | QSY 21 |
| Alexa Fluor 350 | 50 | | | | | | 47 | 50 | | |
| Alexa Fluor 488 | | 64 | 70 | 62 | 60 | 56 | 44 | 49 | 64 | |
| Alexa Fluor 546 | | | | 70 | 71 | 74 | 25 | 29 | 67 | |
| Alexa Fluor 555 | | | | | 47 | 51 | | | 45 | |
| Alexa Fluor 568 | | | | | | 82 | | | 56 | 75 |
| Alexa Fluor 594 | | | | | | 85 | | | | 77 |
| Alexa Fluor 647 | | | | | | | | | | 69 |
| These are exemplary values. Actual values will vary slightly depending upon exact conditions. All dyes available from Molecular Probes, Eugene, OR. | | | | | | | | | | |

Nucleic Acid Polymerases:

Any nucleic acid polymerase that recognizes and incorporates a dual-labeled nucleotide analog according to the invention can be used in nucleic acid sequencing methods according to the invention. Incorporation of dual-labeled nucleotide analogs by a given polymerase is assessed as described above. A non-limiting list of nucleic acid polymerases useful or potentially useful according to the invention is provided in Table 2. The use of variants of these or other polymerases, e.g., variants modified for reduced discrimination against nonconventional nucleotides, or variants modified so as to recognize or accept a particular modified nucleobase moiety, is also specifically contemplated according to the invention. Reaction conditions specific for a given nucleic acid polymerase will be known to those skilled in the art. Exemplary conditions are provided herein in.

Table 2. DNA POLYMERASES BY FAMILY

FAMILY A DNA POLYMERASES

**Bacterial DNA Polymerases**

a) *E. coli* DNA polymerase I
b) Streptococcus pneumoniae DNA polymerase I
c) Thermus aquaticus DNA polymerase I
d) Thermus flavus DNA polymerase I
e) Thermotoga maritima DNA polymerase I

**Bacteriophage DNA Polymerases**

a) T5 DNA polymerase
b) T7 DNA polymerase
c) Spo1 DNA polymerase
d) Spo2 DNA polymerase

Mitochondrial DNA polymerase

Yeast Mitochondrial DNA polymerase II

FAMILY B DNA POLYMERASES

**Bacterial DNA polymerase**

*E. coli* DNA polymerase II

**Bacteriophage DNA polymerase**

a) PRD1 DNA polymerase
b) φ29 DNA polymerase
c) M2 DNA polymerase
d) T4 DNA polymerase

**Archaeal DNA polymerase**

a) Thermococcus litoralis DNA polymerase (Vent)
b) Pyrococcus furiosus DNA polymerase
c) Sulfolobus solfataricus DNA polymerase
d) Thermococcus gorgonarius DNA polymerase
e) Thermococcus species TY

f) Pyrococcus species strain KODI
g) Sulfolobus acidocaldarius
h) Thermococcus species 9°N-7
i) Pyrodictium occultum
j) Methanococcus voltae
k) Desulfurococcus strain TOK (D. Tok Pol)

**Eukaryotic Cell DNA polymerase**

(1) DNA polymerase alpha

a) Human DNA polymerase (alpha)
b) S.cerevisiae DNA polymerase (alpha)
c) S.pombe DNA polymerase I (alpha)
d) Drosophila melanogaster DNA polymerase (alpha)
e) Trypanosoma brucei DNA polymerase (alpha)

(2) DNA polymerase delta

a) Human DNA polymerase (delta)
b) Bovine DNA polymerase (delta)
c) S. cerevisiae DNA polymerase III (delta)
d) S. pombe DNA polymerase III (delta)
e) Plasmodiun falciparum DNA polymerase (delta)

(3) DNA polymerase epsilon

S. cerevisiae DNA polymerase II (epsilon)

(4) Other eukaryotic DNA polymerase

S. cerevisiae DNA polymerase Rev3

**Viral DNA polymerases**

a) Herpes Simplex virus type 1 DNA polymerase
b) Equine herpes virus type 1 DNA polymerase
c) Varicella-Zoster virus DNA polymerase
d) Epstein-Barr virus DNA polymerase
e) Herpesvirus saimiri DNA polymerase
f) Human cytomegalovirus DNA polymerase
g) Murine cytomegalovirus DNA polymerase
h) Human herpes virus type 6 DNA polymerase
i) Channel Catfish virus DNA polymerase
j) Chlorella virus DNA polymerase
k) Fowlpox virus DNA polymerase
l) Vaccinia virus DNA polymerase
m) Choristoneura biennis DNA polymerase
n) Autographa california nuclear polymerase virus (AcMNPV) DNA polymerase
o) Lymantria dispar nuclear polyhedrosis virus DNA polymerase
p) Adenovirus-2 DNA polymerase
q) Adenovirus-7 DNA polymerase
r) Adenovirus-12 DNA polymerase

**Eukaryotic linear DNA plasmid encoded DNA polymerases**

a) S-1 Maize DNA polymerase
b) kalilo neurospora intermedia DNA polymerase

c) pA12 ascobolus immersus DNA polymerase
d) pCLK1 Claviceps purpurea DNA polymerase
e) maranhar neurospora crassa DNA polymerase
f) pEM Agaricus bitorquis DNA polymerase
g) pGKL1 Kluyveromyces lactis DNA polymerase
h) pGKL2 Kluyveromyces lactis DNA polymerase
i) pSKL Saccharomyces kluyveri DNA polymerase

Kits useful according to the invention

The invention encompasses a kit comprising a dual-labeled nucleotide analog useful according to the invention. Kits useful for chain termination reactions can also include a polymerase, or an oligonucleotide primer, or both.

Kits according to the invention can be tailored towards "traditional" chain terminator sequencing or towards minisequencing approaches. In either instance, a kit can contain more than one (e.g., 2, 3, 4 or more) fluorescently labeled dual-labeled nucleotide, wherein each different dual-labeled nucleotide bears a spectrally distinguishable fluorophore.

Kits useful according to the invention will also include packaging materials and instructions necessary for use of the kit. Kits can also include one or more standard templates for evaluating the efficiency and/or fidelity of nucleic acid sequencing reactions.

**EXAMPLES**

Example 1. Synthesis of a dual-labeled nucleotide analog

Nucleotides bearing fluorescent label attached to the nucleobase are available commercially. Alternatively, commercially available modified nucleotides bearing a reactive group (e.g., an amine or thiol group, among others) can be reacted with a fluorescent dye bearing a complementary reactive group. An example of one of the most commonly nucleotide fluorescent labeling approaches is the reaction of a nucleotide bearing an amine, e.g., on an allyl amine or other amine-bearing linker, with the succinimidyl (NHS) ester of the dye. NHS esters of numerous fluorescent dyes are commercially available (e.g., from Molecular Probes, Inc., Eugene, OR) or can be made by one skilled in the art.

A nucleotide bearing a fluorescent label on the nucleobase moiety is labeled with a quencher on the gamma phosphate as follows, to generate a dual-labeled nucleotide according to the invention. The monosodium salt of the fluorescent terminator 2’3’-dideoxyadenosine triphosphate (0.3 mmol) is dissolved in 2 ml of 0.1 M triethylamine bicarbonate (soln. A). This solution is passed over a column of Ag 50W-X8 cation exchange resin equilibrated in soln. A. The column is then washed with 25-50 ml of soln. A and the eluate concentrated to dryness. Five ml of anhydrous methanol is added to the residue, evaporated to dryness, and this procedure repeated twice more and the residue dried under high vacuum for 30 min. The dried residue is dissolved in 5 ml of DMSO, and 3 mmol of dicyclohexyl carbodiimide(DCC) is added and the mixture stirred under nitrogen overnight. One to 3 mmol of dabsyl amine is added and stirred under nitrogen overnight. The solution is diluted with 20 ml of water and acidified to pH 5 with acetic acid, reduced in volume to 5 ml by rotary evaporation and the dual-labeled nucleotide is purified by preparative HPLC and acetonitrile gradient elution.

Example 2. Detection of a nucleotide at a predetermined position using a dual-labeled nucleotide analog

Detection of SNPs is performed by minisequencing using a primer which is fully complementary to the target nucleic acid, such that the primer will anneal to the target so that its 3’ end is hybridized to the target nucleotide immediately adjacent to the nucleotide to be identified. For example, the primer pBA is designed to anneal to pBluescript (A562C) so that the dideoxynucleotide to be incorporated is a cytosine or analog thereof.

pBA 5’- GGATGTGCTGCAAGGCGATT -3’

pBA can be synthesized and HPLC purified by Genset Corporations (La Jolla, CA). 25 μl reactions contained 200 nM fluorescein/DABCYL - dual-labeled ddCTP (that is, a dual labeled nucleotide analog wherein the nucleobase is cytosine), 4 U polymerase, 250 nM pBA, and 200 nM pBluescript in 1 x polymerase reaction buffer. Negative control lacked DNA template (pBluescript). Thermal cycling was performed in the Applied Biosystems Prism 7700 Sequence Detector. Thermal cycling conditions were performed by initial denaturing step at 95°C for 2 minutes, followed by 30 cycles at 95°C for 30 s, 50°C for 1 min, and 57°C for 30 s. The fluorescent intensities were acquired during the annealing/extension phase of the primer extension cycles. The analysis was done using the multicomponent data from the

Applied Biosystems 7700 Sequence Detector. Detection of unquenched fluorescein fluorescence emission is indicative of the incorporation of the dual-labeled ddCTP nucleotide analog, and more particularly, is indicative of that the nucleotide in the predetermined position of the template is a guanine.

<u>Example 3: Generating labeled RNA using a dual-labeled nucleotide according to the invention.</u>

The dual-labeled nucleotides of the invention can be used to generate labeled RNA by in vitro transcription of a DNA template comprising an appropriate promoter. For example, a sequence to be transcribed can be inserted into a vector containing an SP6, T3 or T7 promoter.

The transcript of such a sequence is labeled with T7 polymerase as follows. The vector is linearized with an appropriate restriction enzyme that digests the vector at a single site located downstream of the coding sequence. Following a phenol/chloroform extraction, the DNA is ethanol precipitated, washed in 70% ethanol, dried and resuspended in sterile water. The *in vitro* transcription reaction is performed by incubating the linearized DNA with transcription buffer (40 mM Tris-HCl, pH 8.0, 8 mM $MgCl_2$, 2 mM spermidine, 50 mM NaCl, 30 mM dithiothreitol, RNase inhibitors, 400 μM dual-labeled CTP, 400 μM each of the remaining three ribonucleoside triphosphates ATP, GTP and UTP, and 10 units of T7 RNA polymerase for 30 min at 37°C. The DNA template is then removed by incubation with DNaseI. Fluorescently labeled RNA is ethanol precipitated and re-suspended in buffer appropriate for the hybridization reaction of choice(Ausubel et al., supra). Alternatively, the labeling reaction can be used directly after destruction of the DNA template and inactivation of the DNAse, because fluorescent signal from the unincorporated dual-labeled nucleotides will remain quenched, while the incorporation of the dual-labeled ribonucleotide results in the cleavage of the quencher moiety attached to the polyphosphate. Fluorescent signal will thus only be detected from the incorporated label. It can be helpful to include a proportion of unlabeled CTP in the reaction in order to optimize the yield of labeled RNA. One skilled in the art can determine the proportion empirically without undue experimentation.

<u>Example 4: Generating labeled DNA using a dual-labeled dNTP according to the invention.</u>

DNA probes are labeled using dual-labeled deoxynucleotides according to the invention in primer extension or PCR reactions. Exemplary conditions for PCR labeling are described below.

Dual-labeled dCTP is included in PCR amplification reactions, followed by either purification to remove unincorporated label or direct use, because unincorporated label does not generate a fluorescent signal until a dark quencher is cleaved during incorporation.

Into a PCR amplification vessel is added 5 μL of Taq reaction buffer including Taq polymerase, 20 ng ofpBluescript KS+, 125 ng (each) of reverse and -20 primers, or 125 ng (each) of M13 -20 primer and 066 primer (5'-GCTAATCATGGTCATAGCTGTT-3'), 7.5 μL of a 1mM dGTP-dATP-dTTP solution and 7.5 μL of 500 μM dCTP-500 μM dual-labeled dCTP. Control reactions contain 1 mM dCTP in place of the dCTP/dual-labeled dCTP mixture. Reaction vessels are placed in a thermal cycler and treated according to the following cycling parameters: initial denaturation at 94°C for 45 seconds followed by 28 cycles of 94°C for 45 seconds, annealing at 50°C for 1 minute and extension at 72°C for 1 minute 15 seconds.

Labeled reaction products are ready for use if the quencher used on the dual-labeled nucleotide was a dark quencher, because the quencher is cleaved only when incorporated. Alternatively, labeled reaction products can be purified by, for example, ethanol precipitation, spin column chromatography or preparative gel electrophoresis. The proportion of labeled to unlabeled nucleotide (i.e., dual-labeled dCTP vs dCTP) can be varied as necessary by one of skill in the art in order to achieve optimal labeling.

## OTHER EMBODIMENTS

The foregoing examples demonstrate experiments performed and contemplated by the present inventors in making and carrying out the invention. It is believed that these examples include a disclosure of techniques which serve to both apprise the art of the practice of the invention and to demonstrate its usefulness. It will be appreciated by those of skill in the art that the techniques and embodiments disclosed herein are preferred embodiments only that in general numerous equivalent methods and techniques may be employed to achieve the same result.

## Claims

**1.** A dual labeled nucleotide analog comprising a fluorescent label and a quencher of said fluorescent label, wherein said quencher is attached to a phosphate moiety that is cleaved off when said nucleotide is enzymatically incorporated into a polynucleotide and wherein said nucleotide analog has a general structure selected from the group consisting of:

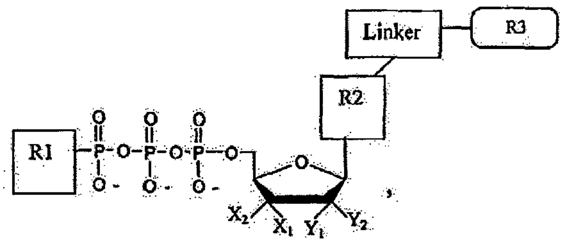

,

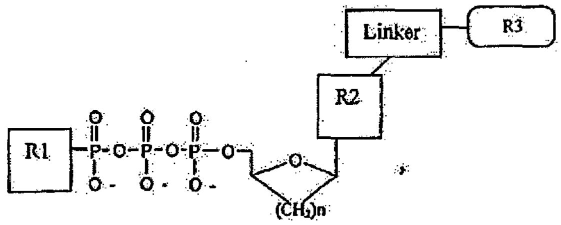

,

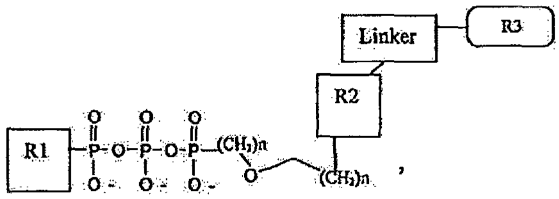

,

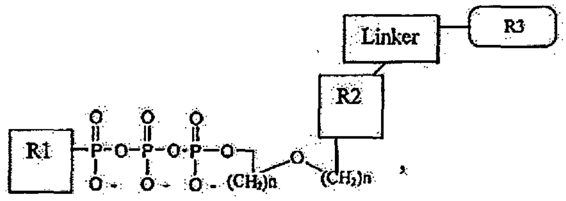

,

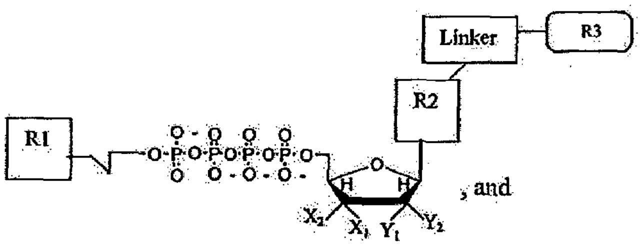

, and

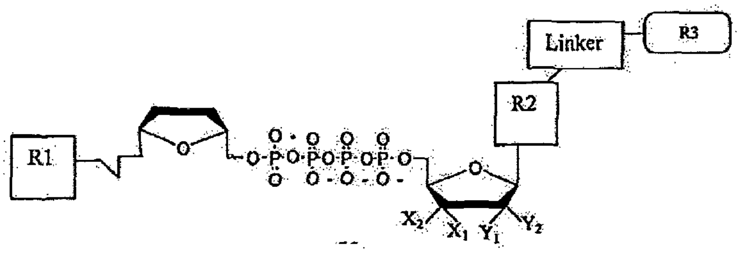

wherein R1 is a quencher moiety;
R2 is a nucleobase;

R3 is a fluorescent moiety that is quenched by said quencher moiety R1; and
n = 1-12; and
X1, X2, Y1, and Y2 are each selected from the group consisting H, OH, F, and $NH_2$.

**2.** The dual labeled nucleotide analog of claim 1, wherein said fluorescent label is attached to the nucleobase of said nucleotide.

**3.** The dual labeled nucleotide analog of claim 1, wherein said fluorescent label is attached to the sugar moiety of said nucleotide.

**4.** The dual labeled nucleotide analog of claim 1, wherein said nucleotide is a nucleotide triphosphate.

**5.** The dual labeled nucleotide analog of claim 1, wherein said nucleotide is a nucleotide tetraphosphate.

**6.** The dual labeled nucleotide analog of claim 5, wherein said quencher is attached to the $\delta$ phosphate of the tetraphosphate moiety.

**7.** The dual labeled nucleotide analog of claim 1, wherein said quencher R1 is a dark quencher.

**8.** The dual labeled nucleotide analog of claim 1, wherein said nucleobase R2 is selected from the group consisting of adenine, cytosine, guanine, thymine, uracil, 7-deazaguanine, and hypoxanthine or an analog thereof.

**9.** The dual labeled nucleotide analog of claim 1, wherein said fluorescent moiety R3 is selected from the group consisting of R110, TAMRA, R6G and ROX.

**10.** The dual labeled nucleotide analog of claim 1, wherein said linker is attached to said nucleobase at the N-4 or C-5 position when said nucleobase is a pyrimidine, or at the N-6, C-8, or C(N)-7 position when said nucleobase is a purine.

**11.** The dual labeled nucleotide analog of claim 1, wherein said nucleotide comprises a sugar moiety selected from the group consisting of ribofuranosyl, 2'-deoxyribofuranosyl, 2', 3'-dideoxyribofuranosyl, phosphonomethoxyethyl, 2-oxyethoxymethyl, 2-hydroxymethoxymethyl, 3-pentenyl, oxetan, and pyran.

**12.** A method of synthesizing a polynucleotide, the method comprising contacting a nucleic acid polymerase enzyme with the dual labeled nucleotide analog according to claim 1, under conditions permitting the extension of a nucleic acid primer annealed to a template nucleic acid, wherein said dual labeled nucleotide analog is thereby incorporated into said nucleic acid primer.

**13.** The method of claim 12, wherein said contacting results in chain termination.

**14.** The method of claim 13, wherein said contacting permits the determination of nucleic acid sequence information about said template nucleic acid.

**15.** A method of labelling a polynucleotide, the method comprising contacting a nucleic acid polymerase enzyme with the dual labeled nucleotide analog according to claim 1, under conditions permitting the extension of a nucleic acid primer annealed to a template nucleic acid, whereby said dual-labeled nucleotide analog is incorporated into said nucleic acid primer, thereby labeling said polynucleotide.

**16.** A method of determining sequence information about a template polynucleotide, the method comprising

(a) annealing an oligonucleotide primer to a template polynucleotide to generate an annealed primer;
(b) contacting said annealed primer and said template of step (a) with a nucleic acid polymerase enzyme in the presence of the dual labeled nucleotide analog according to claim 1, under conditions to permit the extension of said primer by said nucleic acid polymerase enzyme; and
(c) detecting the incorporation of said dual-labeled nucleotide analog into said primer, wherein said incorporation determines sequence information about said template polynucleotide.

**17.** The method of claim 12, claim 15 or claim 16, wherein said nucleotide is selected from the group consisting of a nucleotide di-, tri-, or tetra-phosphate.

18. The method of claim 12, claim 15 or claim 16, wherein said nucleotide is a nucleotide thiophosphate, and said quencher is linked to the γ-phosphate of said thiophosphate.

19. The method of claim 12, claim 15 or claim 16, wherein said nucleotide comprises a sugar moiety selected from the group consisting of ribofuranosyl, 2'-deoxyribofuranosyl, 2', 3'-dideoxyribofuranosyl, phosphonomethoxyethyl, 2-oxyethoxymethyl, 2-hydroxymethoxymethyl, 3-pentenyl, oxetan, and pyran.

20. The method of claim 12, claim 15 or claim 16, wherein said quencher is a dark quencher.

21. The method of claim 12, claim 15 or claim 16, wherein said nucleotide comprises a nucleobase selected from the group consisting of adenine, cytosine, guanine, thymine, uracil, 7-deazaguanine, and hypoxanthine, or an analog thereof.

22. The method of claim 12, claim 15 or claim 16, wherein said fluorescent label is selected from the group consisting of R110, TAMRA, R6G and ROX.

23. The method of claim 16, wherein said method is performed on a solid support.

24. The method of claim 16, wherein said incorporation of said nucleotide into said primer results in cleavage of said quencher from said nucleotide.

25. The method of claim 16, wherein said detection step comprises contacting said nucleotide with light of a wavelength that is within the excitation spectrum of said fluorescent moiety and detecting the resulting emission of fluorescent light from said nucleotide.

26. A kit comprising the dual labeled nucleotide analog of claim 1 and a nucleic acid polymerase and/or an oligonucleotide primer.

## Patentansprüche

1. Dual-markiertes Nukleotidanalogon, welches eine fluoreszierende Markierung und einen Quencher der fluoreszierenden Markierung umfasst, wobei der Quencher an eine Phosphateinheit angeheftet ist, die abgespalten wird, wenn das Nukleotid enzymatisch in ein Polynukleotid eingeschlossen wird, und wobei das Nukleotidanalogon eine allgemeine Struktur aufweist, die aus der Gruppe bestehend aus Folgenden ausgewählt ist:

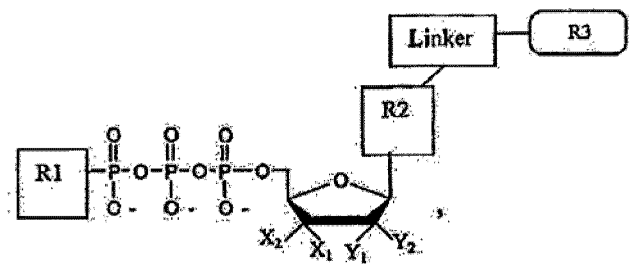

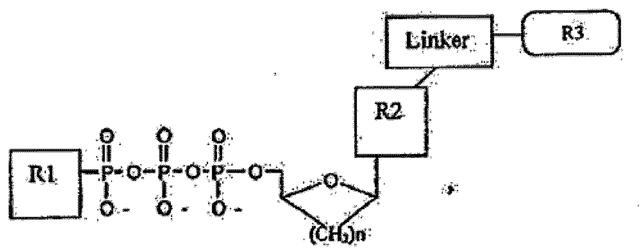

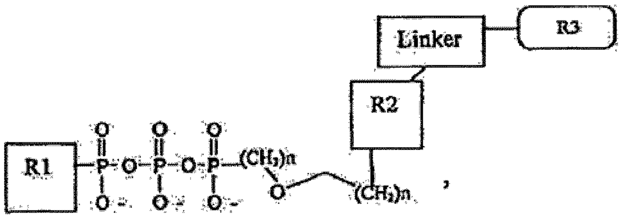

,

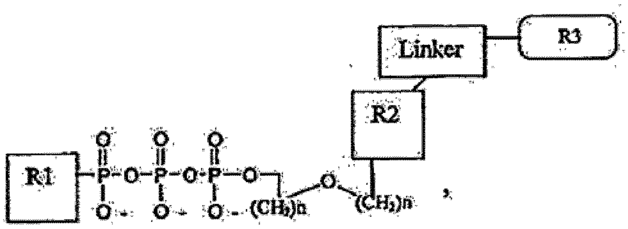

,

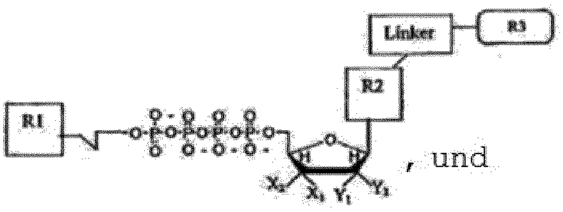

, und

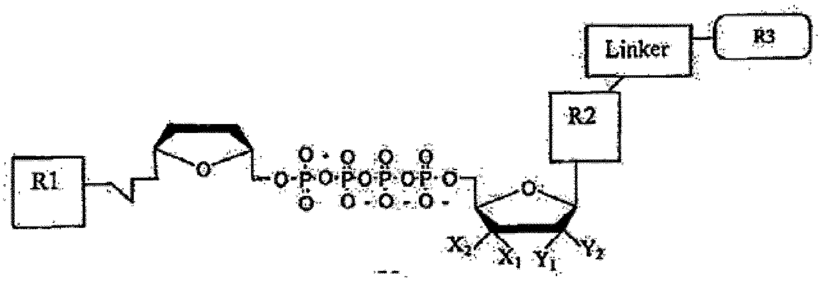

,

wobei R1 eine Quenchereinheit ist;
R2 eine Nukleobase ist;
R3 eine fluoreszierende Einheit ist, die durch die Quenchereinheit R1 gequencht wird; und
n=1-12; und
X1, X2, Y1 und Y2 jeweils ausgewählt sind aus der Gruppe bestehend aus H, OH, F und $NH_2$.

**2.** Dual-markiertes Nukleotidanalogon nach Anspruch 1, wobei die fluoreszierende Markierung an der Nukleobase des Nukleotids angehaftet ist.

**3.** Dual-markiertes Nukleotidanalogon nach Anspruch 1, wobei die fluoreszierende Markierung an der Zuckereinheit des Nukleotids angehaftet ist.

**4.** Dual-markiertes Nukleotidanalogon nach Anspruch 1, wobei das Nukleotid ein Nukleotidtriphosphat ist.

**5.** Dual-markiertes Nukleotidanalogon nach Anspruch 1, wobei das Nukleotid ein Nukleotidtetraphosphat ist.

**6.** Dual-markiertes Nukleotidanalogon nach Anspruch 5, wobei der Quencher an dem δ-Phosphat der Tetraphosphateinheit angehaftet ist.

**7.** Dual-markiertes Nukleotidanalogon nach Anspruch 1, wobei der Quencher R1 ein Dunkelquencher ist.

**8.** Dual-markiertes Nukleotidanalogon nach Anspruch 1, wobei die Nukleobase R2 ausgewählt ist aus der Gruppe bestehend aus Adenin, Cytosin, Guanin, Thymin, Uracil, 7-Deazaguanin und Hypoxanthin oder einem Analogon davon.

**9.** Dual-markiertes Nukleotidanalogon nach Anspruch 1, wobei die fluoreszierende Einheit R3 ausgewählt ist aus der Gruppe bestehend aus R110, TAMRA, R6G und ROX.

**10.** Dual-markiertes Nukleotidanalogon nach Anspruch 1, wobei der Linker an der N-4- oder C-5-Position an die Nukleobase angeheftet ist, wenn die Nukleobase ein Pyrimidin ist, oder an der N-6-, C-8-oder C(N)-7-Position, wenn die Nukleobase ein Purin ist.

**11.** Dual-markiertes Nukleotidanalogon nach Anspruch 1, wobei das Nukleotid eine Zuckereinheit ausgewählt aus der Gruppe bestehend aus Ribofuranosyl, 2'-Deoxyribofuranosyl, 2',3'-Dideoxyribo-furanosyl, Phosphonomethoxyethyl, 2-Oxyethoxymethyl, 2-Hydroxymethoxymethyl, 3-Pentenyl, Oxetan und Pyran umfasst.

**12.** Verfahren zum Synthetisieren eines Polynukleotids, wobei das Verfahren das in Kontakt bringen eines Nukleinsäure-Polymerase-Enzyms mit dem dual-markierten Nukleotidanalogon nach Anspruch 1 unter Bedingungen, welche die Erweiterung eines Nukleinsäureprimers gebunden an eine Template-Nukleinsäure gestatten, umfasst, wobei das dual-markierte Nukleotidanalogon dadurch in den Nukleinsäureprimer eingeschlossen wird.

**13.** Verfahren nach Anspruch 12, wobei das in Kontakt bringen in Kettenabbruch resultiert.

**14.** Verfahren nach Anspruch 13, wobei das in Kontakt bringen die Bestimmung von Nukleinsäuresequenz-Informationen über die Template-Nukleinsäure gestattet.

**15.** Verfahren zum Markieren eines Polynukleotids, wobei das Verfahren das in Kontakt bringen eines Nukleinsäure-Polymerase-Enzyms mit dem dual-markierten Nukleotidanalogon nach Anspruch 1 unter Bedingungen, welche die Erweiterung eines Nukleinsäureprimers gebunden an eine Template-Nukleinsäure gestatten, umfasst, wodurch das dual-markierte Nukleotidanalogon in den Nukleinsäureprimer eingeschlossen wird, wodurch das Polynukleotid markiert wird.

**16.** Verfahren zum Bestimmen von Sequenzinformationen über ein Template-Polynukleotid, wobei das Verfahren Folgendes umfasst:

(a) Binden eines Oligonukleotidprimers an ein Template-Polynukleotid zum Erzeugen eines gebundenen Primers;
(b) in Kontakt bringen des gebundenen Primers und des Templates aus Schritt (a) mit einem Nukleinsäure-Polymerase-Enzym in Gegenwart des dual-markierten Nukleotidanalogons nach Anspruch 1 unter Bedingungen, welche ausreichend sind, um die Erweiterung des Primers durch das Nukleinsäure-Polymerase-Enzym zu gestatten; und
(c) Nachweisen des Einschlusses des dual-markierten Nukleotidanalogons in den Primer, wobei der Einschluss Sequenzinformationen über das Template-Polynukleotid bestimmt.

**17.** Verfahren nach Anspruch 12, Anspruch 15 oder Anspruch 16, wobei das Nukleotid ausgewählt ist aus der Gruppe bestehend aus einem Nukleotid-Di-, -Tri- oder -Tetraphosphat.

**18.** Verfahren nach Anspruch 12, Anspruch 15 oder Anspruch 16, wobei das Nukleotid ein Nukleotidtriphosphat ist und der Quencher an das $\gamma$-Phosphat des Triphosphates gebunden ist.

**19.** Verfahren nach Anspruch 12, Anspruch 15 oder Anspruch 16, wobei das Nukleotid eine Zuckereinheit ausgewählt aus der Gruppe bestehend aus Ribofuranosyl, 2'-Deoxyribofuranosyl, 2',3'-Dideoxyribofuranosyl, Phosphonomethoxyethyl, 2-Oxyethoxymethyl, 2-Hydroxymethoxymethyl, 3-Pentenyl, Oxetan und Pyran umfasst.

**20.** Verfahren nach Anspruch 12, Anspruch 15 oder Anspruch 16, wobei der Quencher ein Dunkelquencher ist.

**21.** Verfahren nach Anspruch 12, Anspruch 15 oder Anspruch 16, wobei das Nukleotid eine Nukleobase ausgewählt aus der Gruppe bestehend aus Adenin, Cytosin, Guanin, Thymin, Uracil, 7-Deazaguanin und Hypoxanthin oder ein Analogon davon umfasst.

**22.** Verfahren nach Anspruch 12, Anspruch 15 oder Anspruch 16, wobei die fluoreszierende Markierung ausgewählt ist aus der Gruppe bestehend aus R110, TAMRA, R6G und ROX.

**23.** Verfahren nach Anspruch 16, wobei das Verfahren auf einem festen Träger durchgeführt wird.

**24.** Verfahren nach Anspruch 16, wobei der Einschluss des Nukleotids in den Primer in einer Abspaltung des Quenchers von dem Nukleotid resultiert.

**25.** Verfahren nach Anspruch 16, wobei der Nachweisschritt das in Kontakt bringen des Nukleotids mit Licht einer Wellenlänge, die innerhalb des Anregungsspektrums der fluoreszierenden Einheit liegt, und das Nachweisen der resultierenden Emission von fluoreszierendem Licht aus dem Nukleotid umfasst.

**26.** Kit, welches das dual-markierte Nukleotidanalogon nach Anspruch 1 und eine Nukleinsäure-Polymerase und/oder einen Oligonukleotidprimer umfasst.

## Revendications

**1.** Analogue de nucléotide à double marquage comprenant un marqueur fluorescent et un agent d'extinction dudit marqueur fluorescent, dans lequel ledit agent d'extinction est fixé à une fraction phosphate qui est clivée lorsque ledit nucléotide est incorporé par voie enzymatique à un polynucléotide et dans lequel ledit analogue de nucléotide a une structure générale choisie dans le groupe constitué des suivantes :

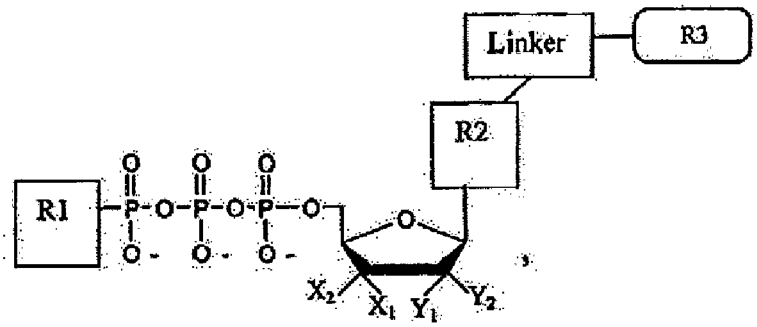

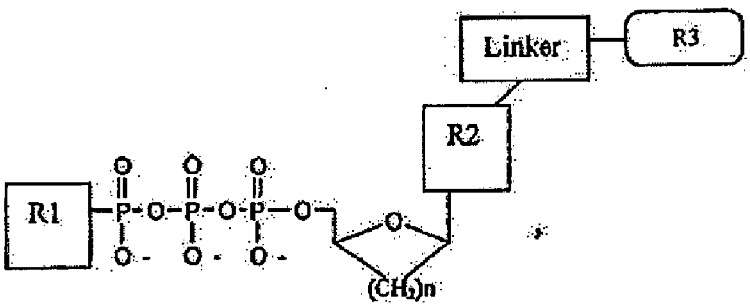

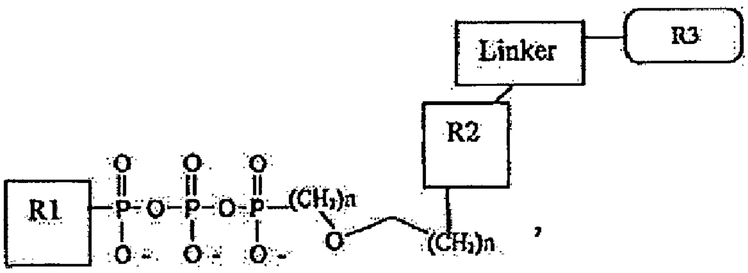

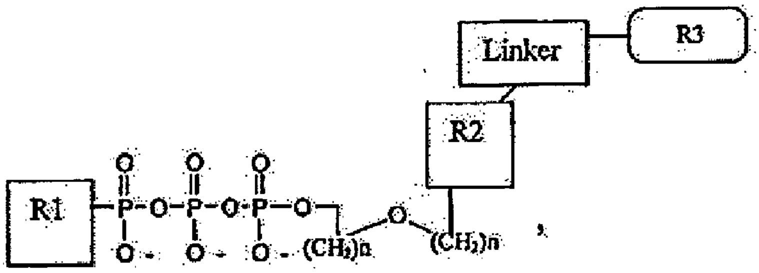

,

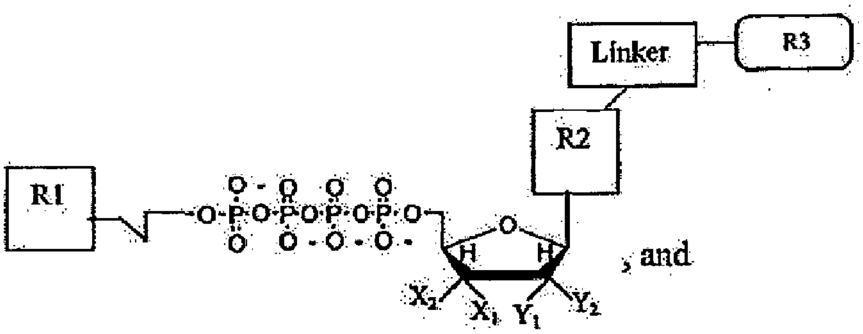

, and

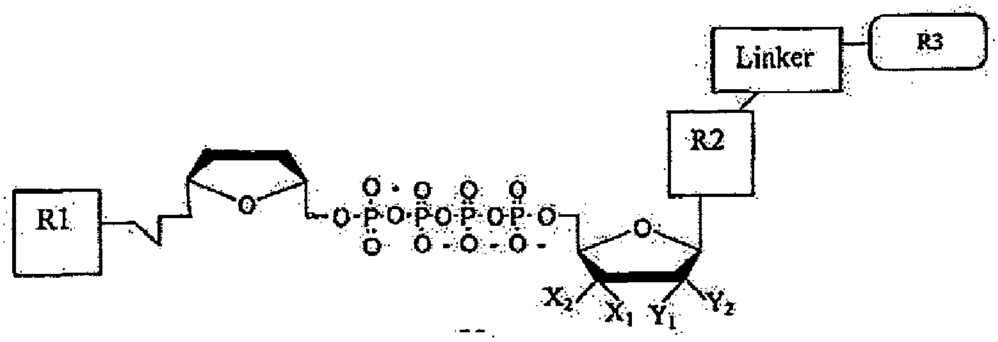

[Linker = Lieur ; and = et]
dans lesquelles R1 est une fraction d'agent d'extinction ;
R2 est une nucléobase ;
R3 est une fraction fluorescente qui est éteinte par ladite fraction d'agent d'extinction R1 ; et
n = 1 à 12 ; et
X1, X2, Y1 et Y2 sont chacun choisis dans le groupe constitué de H, OH, F et $NH_2$.

2. Analogue de nucléotide à double marquage selon la revendication 1, dans lequel ledit marqueur fluorescent est fixé à la nucléobase dudit nucléotide.

3. Analogue de nucléotide à double marquage selon la revendication 1, dans lequel ledit marqueur fluorescent est fixé à la fraction sucre dudit nucléotide.

4. Analogue de nucléotide à double marquage selon la revendication 1, dans lequel ledit nucléotide est un nucléotide triphosphate.

5. Analogue de nucléotide à double marquage selon la revendication 1, dans lequel ledit nucléotide est un nucléotide tétraphosphate.

6. Analogue de nucléotide à double marquage selon la revendication 5, dans lequel ledit agent d'extinction est fixé au phosphate ∂ de la fraction tétraphosphate.

**7.** Analogue de nucléotide à double marquage selon la revendication 1, dans lequel ledit agent d'extinction R1 est un agent d'extinction sombre.

**8.** Analogue de nucléotide à double marquage selon la revendication 1, dans ladite nucléobase R2 est choisie dans le groupe constitué de l'adénine, de la cytosine, de la guanine, de la thymine, de l'uracile, de la 7-désazaguanine et de l'hypoxanthine ou d'un de leurs analogues.

**9.** Analogue de nucléotide à double marquage selon la revendication 1, dans lequel ladite fraction fluorescente R3 est choisie dans le groupe constitué de R110, TAMRA, R6G et ROX.

**10.** Analogue de nucléotide à double marquage selon la revendication 1, dans lequel ledit lieur est fixé à ladite nucléobase en position N-4 ou C-5 lorsque ladite nucléobase est une pyrimidine ou en position N-6, C-8 ou C(N)-7 lorsque ladite nucléobase est une purine.

**11.** Analogue de nucléotide à double marquage selon la revendication 1, dans lequel ledit nucléotide comprend une fraction sucre choisie dans le groupe constitué des fractions ribofuranosyle, 2'-désoxyribofuranosyle, 2',3'-didésoxyribo-furanosyle, phosphonométhoxyéthyle, 2-oxyéthoxy-méthyle, 2-hydroxyméthoxyméthyle, 3-penténylе, oxétane et pyrane.

**12.** Procédé de synthèse d'un polynucléotide, le procédé comprenant la mise en contact d'une enzyme polymérase d'acide nucléique avec l'analogue de nucléotide à double marquage selon la revendication 1 dans des conditions permettant l'extension d'une amorce d'acide nucléique hybridée à un acide nucléique matrice, dans lequel ledit analogue de nucléotide à double marquage est ainsi incorporé à ladite amorce d'acide nucléique.

**13.** Procédé selon la revendication 12, dans lequel ladite mise en contact entraîne une terminaison de chaîne.

**14.** Procédé selon la revendication 13, dans lequel ladite mise en contact permet la détermination d'informations de la séquence d'acide nucléique sur ledit acide nucléique matrice.

**15.** Procédé de marquage d'un polynucléotide, le procédé comprenant la mise en contact d'une enzyme polymérase d'acide nucléique avec un analogue de nucléotide à double marquage selon la revendication 1 dans des conditions permettant l'extension d'une amorce d'acide nucléique hybridée à un acide nucléique matrice, si bien que ledit analogue de nucléotide à double marquage est incorporé à ladite amorce d'acide nucléique, en marquant de la sorte ledit polynucléotide.

**16.** Procédé de détermination d'informations de séquence sur un polynucléotide matrice, le procédé comprenant les étapes consistant à :

(a) hybrider une amorce d'oligonucléotide à un polynucléotide matrice pour générer une amorce hybridée ;
(b) mettre en contact ladite amorce hybridée et ladite matrice de l'étape (a) avec une enzyme polymérase d'acide nucléique en présence de l'analogue de nucléotide à double marquage selon la revendication 1 dans des conditions suffisantes pour permettre l'extension de ladite amorce par ladite enzyme polymérase d'acide nucléique ; et
(c) détecter l'incorporation dudit analogue de nucléotide à double marquage à ladite amorce, dans lequel ladite incorporation détermine des informations de séquence sur ledit polynucléotide matrice.

**17.** Procédé selon la revendication 12, la revendication 15 ou la revendication 16, dans lequel ledit nucléotide est choisi dans le groupe constitué d'un nucléotide di-, tri- ou tétraphosphate.

**18.** Procédé selon la revendication 12, la revendication 15 ou la revendication 16, dans lequel ledit nucléotide est un nucléotide thiophosphate et ledit agent d'extinction est lié au phosphate y dudit thiophosphate.

**19.** Procédé selon la revendication 12, la revendication 15 ou la revendication 16, dans lequel ledit nucléotide comprend une fraction sucre choisie dans le groupe constitué des fractions ribofuranosyle, 2'-désoxyribofuranosyle, 2',3'-didésoxyribofuranosyle, phosphonométhoxy-éthyle, 2-oxyéthoxyméthyle, 2-hydroxyméthoxy-méthyle, 3-penténylе, oxétane et pyrane.

**20.** Procédé selon la revendication 12, la revendication 15 ou la revendication 16, dans lequel ledit agent d'extinction

est un agent d'extinction sombre.

**21.** Procédé selon la revendication 12, la revendication 15 ou la revendication 16, dans lequel ledit nucléotide comprend une nucléobase choisie dans le groupe constitué de l'adénine, de la cytosine, de la guanine, de la thymine, de l'uracile, de la 7-désazaguanine et de l'hypoxanthine ou de l'une de leurs analogues.

**22.** Procédé selon la revendication 12, la revendication 15 ou la revendication 16, dans lequel ledit marqueur fluorescent est choisi dans le groupe constitué de R110, TAMRA, R6G et ROX.

**23.** Procédé selon la revendication 16, dans lequel ledit procédé est effectué sur un support solide.

**24.** Procédé selon la revendication 16, dans lequel ladite incorporation dudit nucléotide à ladite amorce entraîne le clivage dudit agent d'extinction dudit nucléotide.

**25.** Procédé selon la revendication 16, dans lequel ladite étape de détection comprend la mise en contact dudit nucléotide avec de la lumière d'une longueur d'onde qui se situe dans le spectre d'excitation de ladite fraction fluorescente et la détection de l'émission de lumière fluorescente dudit nucléotide.

**26.** Kit comprenant l'analogue de nucléotide à double marquage selon la revendication 1 et une polymérase d'acide nucléique et/ou une amorce d'oligonucléotide.

# Figure 1

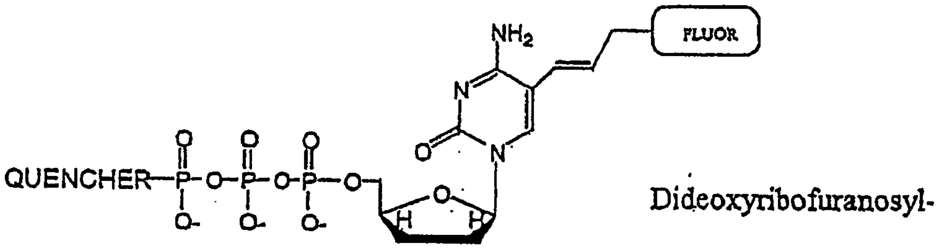

Dideoxyribofuranosyl-

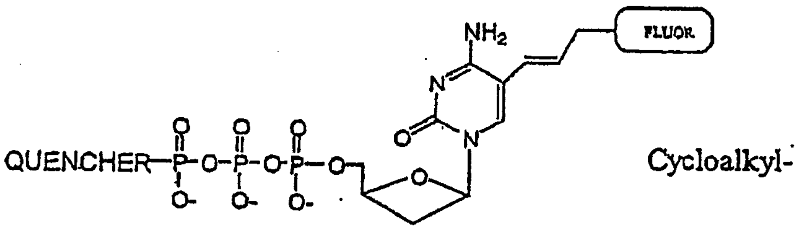

Cycloalkyl-

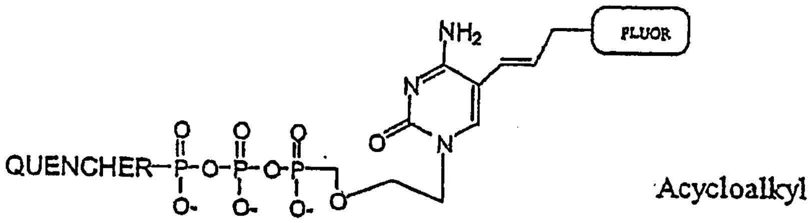

Acycloalkyl (PME-)

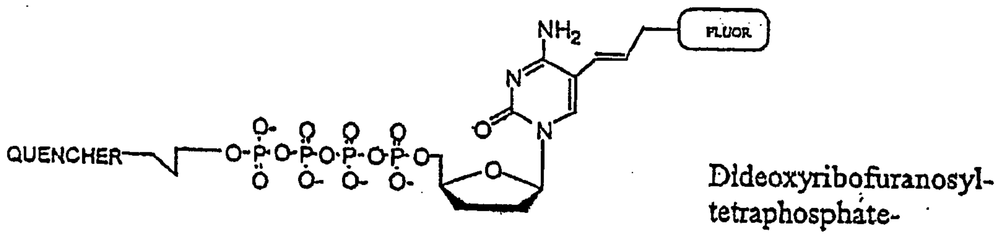

Dideoxyribofuranosyl-
tetraphosphate-

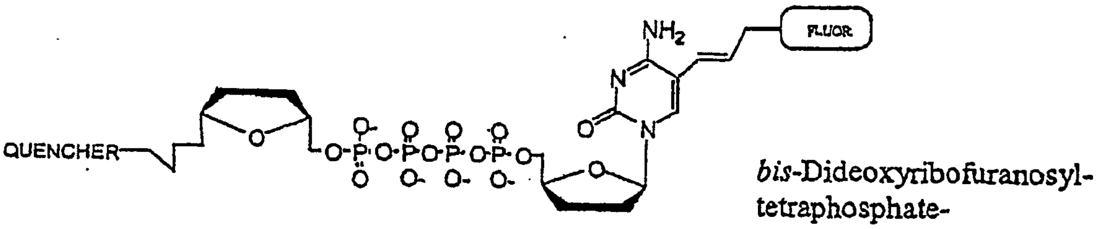

*bis*-Dideoxyribofuranosyl-
tetraphosphate-

## Figure 2

Exemplary Acyclic Nucleoside Analogs

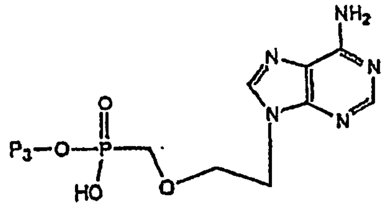

1b

PMEApp (triphosphate)

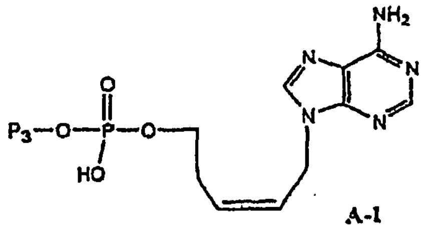

A-1

3-pentenyl

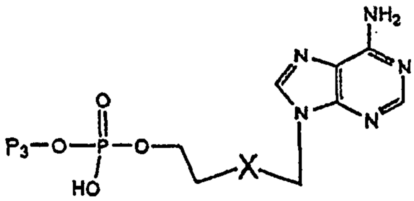

B-1 X=O
B-2 X=$CH_2$

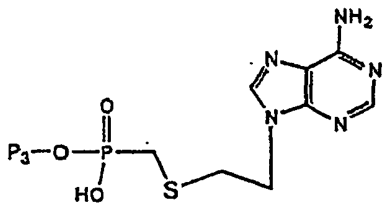

B-3

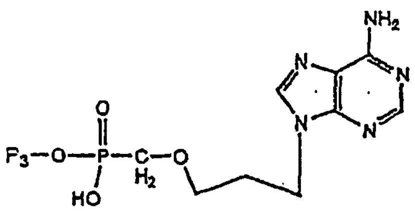

B-4

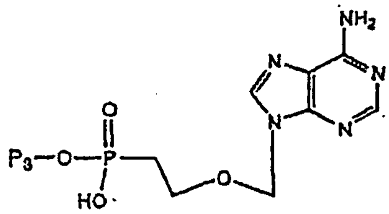

B-5

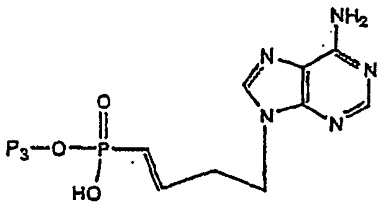

B-6

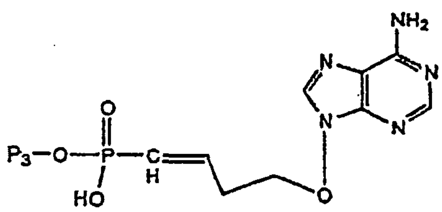

B-7

Figure 3.
Examples of Dual-Labeled NTPs
with Dye Attached to the "Sugar" Moiety

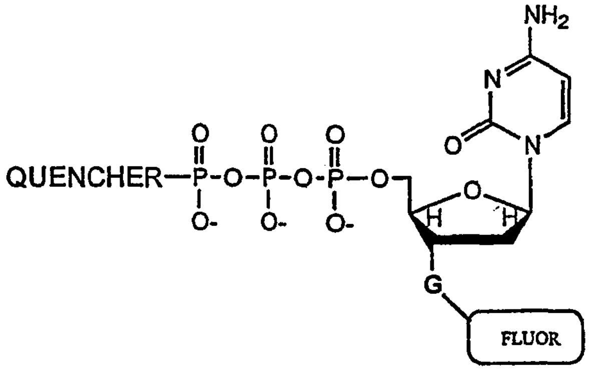

G = O, NH, S

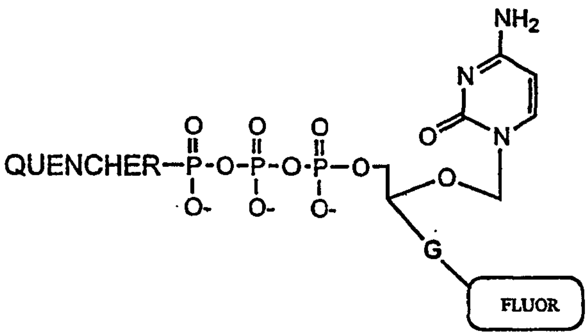

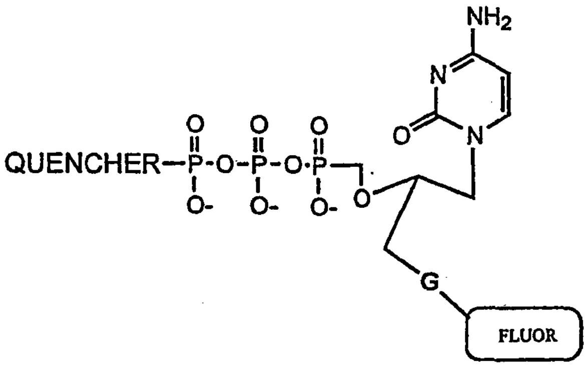

Figure 4.
Examples of Dual-Labeled NTPs
with Dye Attached to the α-Phosphate Moiety

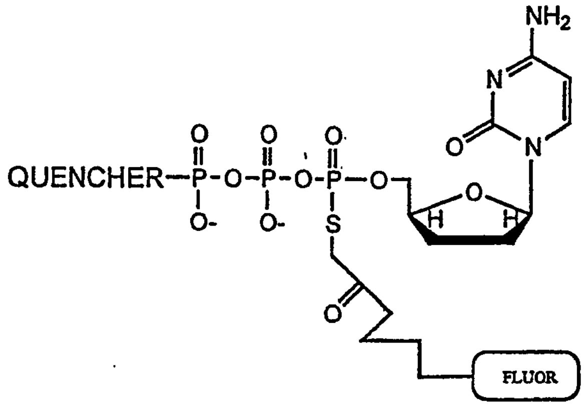

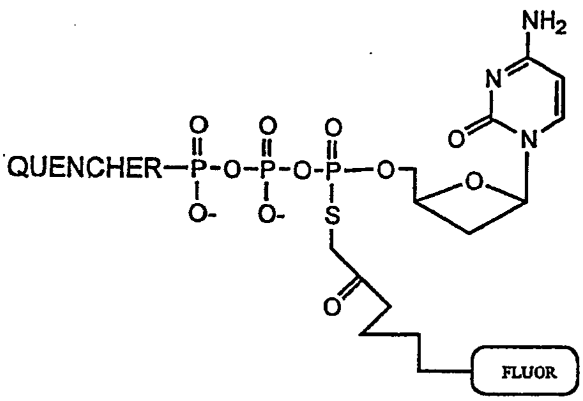

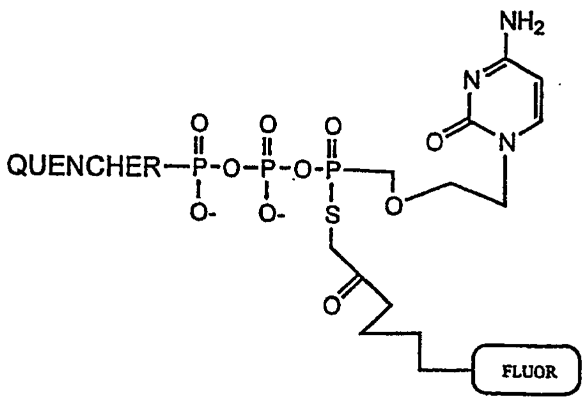

Figure 5

Structure of Dual Labeled 7-deaza-Guanosine

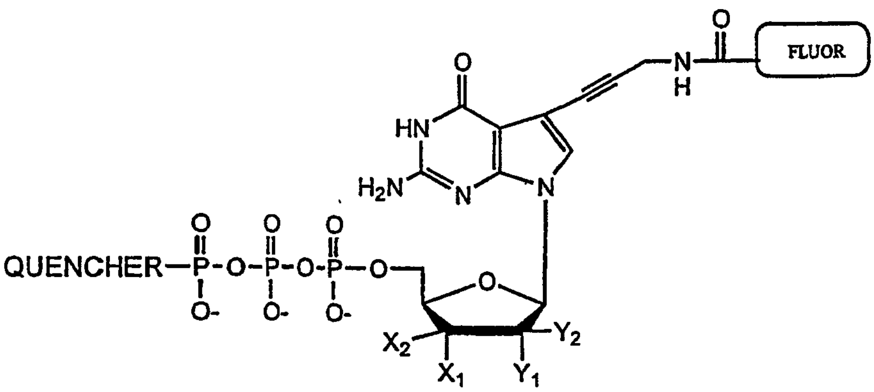

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 20010018184 A, Williams
- US 6329205 B
- WO 0186001 A
- US 6001983 A
- US 3996345 A, Ullman
- US 4351760 A, Khanna
- US 5476928 A
- US 5958691 A
- US 5151507 A
- US 5608063 A
- US 5047519 A
- US 5093232 A
- US 5679785 A
- US 5359067 A
- US 4757141 A, Fung
- US 6016195 A
- US 5866348 A
- US 10407710 A
- US 60400558 B
- US 60372351 B


### Non-patent literature cited in the description


- **ROSENBLUM et al.** *Nucleic Acids Research,* 1997, vol. 25, 4500
- *Bioorg. Med. Chem. Lett.,* 1997, vol. 7, 3013-3016
- *Nucl. Acids Res.,* 1999, vol. 27, 1271-1274
- *Nucleosides and Nucleotides,* 1993, vol. 12, 83-93
- **KORNBERG ; BAKER.** DNA Replication. Freeman, San Francisco, 1992
- **SCHEIT.** Nucleotide Analogs. John Wiley, 1980
- **SANGER et al.** *J. Mol. Biol.,* 1975, vol. 94, 441
- **BERLNAN.** Handbook of Fluorescence Spectra of Aromatic Molecules. Academic Press, 1971
- **GRIFFITHS.** Colour and Constitution of Organic Molecules. Academic Press, 1976
- Indicators. Pergamon Press, 1972
- **HAUGLAND.** Handbook of Fluorescent Probes and Research Chemicals. Molecular Probes, 1992
- **PRINGSHEIM.** Fluorescence and Phosphorescence. Interscience Publishers, 1949
- *Helvetica. Chim. Acta,* 1999, vol. 82, 1311-1323
- *J Med. Chem,* 1980, vol. 23, 569
- *Nucleosides, Nucleotides and Nucleic Acids,* 1997, vol. 16, 107-114
- **RUSSIAN.** *Chem. Rev.,* 1999, vol. 68, 483-504
- *J. Chem. Soc. Chem. Commun.,* 1994, 1997-8
- *Nucl. Acids Res.,* 2001, vol. 29, 1565-1573
- *Nucleosides, Nucleotides & Nucleic Acids,* 1999, vol. 18, 979-980
- *Bull. Chem. Soc. Jpn,* 1995, vol. 68, 1981-1987
- **R.S. SARFATI.** *Proc. Nat. Acad. Sci USA,* 1995, vol. 92, 10859-10863
- **ENGELS.** *Bioorg. Med. Chem Lett.,* 1994, vol. 4, 1975-1978
- **HOLY ; ROSENBERG.** *Collect. Czech. Chem. Commun.,* 1987, vol. 52, 2775
- **HOLY ; ROSENBERG.** *Collect. Czech. Chem. Commun.,* 1987, vol. 52, 2801
- **STARRETT et al.** *J. Med. Chem.,* 1994, vol. 37, 1857-1864
- **MOFFATT.** *Can. J. Chem.,* 1964, vol. 42, 599
- *J. Heterocycl. Chem.,* 2000, vol. 37, 1187-91
- *Collect. Czech. Chem. Commun.,* 1989, vol. 54, 2190
- *J. Med. Chem.,* 1999, vol. 42, 2064-2086
- *Can.J. Chem.,* 1964, vol. 42, 599
- *Collect. Czech. Chem. Commun.,* 1987, vol. 52, 2801-9
- *Nucl. Acids. Res.,* 1973, 19-34
- *Collect. Czech. Chem. Commun.,* 1989, vol. 54, 2190-2209
- *J. Org. Chem.,* 1989, vol. 54, 3420-3422
- **BALOW, G.** *Nucleosides, Nucleotides,* 1977, vol. 16, 941-944
- **KAO et al.** *Virology,* 2000, vol. 274, 429-437
- **RYCHLIK et al.** *Biochim. Biophys. Acta,* 1982, vol. 698, 116-127
- **BRENNER.** *J. Biol Chem,* 2000, vol. 275, 4555-4560
- Oligonucleotides and Analogues: A Practical Approach. IRL Press, 1991
- **ZUCKERMAN et al.** *Nucleic Acids Research,* 1987, vol. 15, 5305-5321

- **SHARMA et al.** *Nucleic Acids Research,* 1991, vol. 19, 3019
- **GIUSTI et al.** *PCR Methods and Applications,* 1993, vol. 2, 223-227
- **BLAKLEY.** *BioChemistry,* 1975, vol. 14, 4804-4812
- **NELSON et al.** *Nucleic Acids Research,* 1989, vol. 17, 7187-7194
- **KRAYAVSKY et al.** *J. Biol Chem,* 1996, vol. 271, 24389-24394
- **FELICIA et al.** *Arch. Biochem Biophys.,* 1986, vol. 246, 564-571
- **SANGER et al.** *Proc. Natl. Acad. Sci. U.S.A.,* 1977, vol. 74, 5463-5467
- **PROBER et al.** *Science,* 1987, vol. 238, 336-341
- **SYVANEN et al.** *Genomics,* vol. 8, 684-692
- **MAKRIDAKIS ; REICHARDT.** *Biotechniques,* 2001, vol. 6, 1374-1380
- **HUBER et al.** *Anal. Biochem.,* 2001, vol. 299, 24-30
- **SHAPERO et al.** *Genome Res.,* 2001, vol. 11, 1926-1934

SEQUENCE LISTING

<110> Anderson, Jack D.
Braman, Jeffrey C.

<120> Dual-Labeled Nucleotides

<130> 25436/2212

<140> 10/407,710
<141> 2003-04-04

<150> US 60/400,558
<151> 2002-08-02

<150> US 60/372,351
<151> 2002-04-12

<160> 2

<170> PatentIn version 3.1

<210> 1
<211> 20
<212> DNA
<213> Artificial

<220>
<221> misc_feature
<222> (1)..(20)
<223> synthetic oligonucleotide primer designed to anneal to pBluescrip t (A256C).

<400> 1
ggatgtgctg caaggcgatt 20

<210> 2
<211> 22
<212> DNA
<213> Artificial

<220>
<221> misc_feature
<222> (1)..(22)
<223> Synthetic oligonucleotide primer 066 for amplification of M13 pha ge sequences.

<400> 2
gctaatcatg gtcatagctg tt 22